# EUROPEAN PATENT SPECIFICATION

(11) **EP 1 613 661 B1**
(45) Date of publication and mention of the grant of the patent: **29.06.2011**
(21) Application number: 04725746.4
(22) Date of filing: 05.04.2004
(51) Int. Cl.: C07K 19/00, C12N 15/62

(54) **PEPTABODY FOR CANCER TREATMENT**
PEPTABODY FÜR KREBSBEHANDLUNG
PEPTABODY POUR LE TRAITEMENT DU CANCER

(30) Priority: 04.04.2003 US 460490 P
(43) Date of publication of application: 11.01.2006
(73) Proprietor: Université de Lausanne, 1005 Lausanne (CH)
(72) Inventor: DEPERTHES, David, 1201 Genève (CH); CLOUTIER, Sylvain, G1H-3Y8 Québec (CA); MACH, Jean-Pierre, CH-1010 Lausanne (CH); HOLLER, Nils, 3063 Ittingen (CH); FATTAH, Omar, 01170 Gex (FR)
(74) Representative: KATZAROV S.A.
(86) International application number: PCT/IB2004/001049
(87) International publication number: WO 2004/087766

(56) References cited:
- WO-A-02/06469
- WO-A-94/18345
- WO-A-98/18943
- WO-A-02/066628
- US-A- 5 571 507
- US-B1- 6 348 568
- US-B1- 6 350 860
- HOUIMEL MEHDI ET AL: "Selection of peptides and synthesis of pentameric peptabody molecules reacting specifically with ErbB-2 receptor" INTERNATIONAL JOURNAL OF CANCER, vol. 92, no. 5, 1 June 2001 (2001-06-01), pages 748-755, XP009034136 ISSN: 0020-7136
- DEPERTHES DAVID LUC ET AL: "Effect of peptabody anti Epidermal Growth Factor receptors on prostate cancer cell lines proliferation" EUROPEAN UROLOGY SUPPLEMENTS, vol. 1, no. 1, January 2002 (2002-01), page 31, XP009034146 & XVIITH CONGRESS OF THE EUROPEAN ASSOCIATION OF UROLOGY; BIRMINGHAM, ENGLAND, UK; FEBRUARY 23-26, 2002 ISSN: 1569-9056
- CAMPBELL I D ET AL: "STRUCTURE-FUNCTION RELATIONSHIPS IN EPIDERMAL GROWTH FACTOR EGF AND TRANSFORMING GROWTH FACTOR-ALPHA TGF-ALPHA" BIOCHEMICAL PHARMACOLOGY, vol. 40, no. 1, 1990, pages 35-40, XP002289670 & FOURTH BIOCHEMICAL PHARMACOLOGY SYMPOSIUM ON NMR METHODS FOR ELUCIDATING MACROMOLECULE-LIGAND INTERA ISSN: 0006-2952 cited in the application
- STORTELERS CATELIJNE ET AL: "Epidermal growth factor contains both positive and negative determinants for interaction with ErbB-2/ErbB-3 heterodimers" BIOCHEMISTRY, vol. 41, no. 13, 2 April 2002 (2002-04-02), pages 4292-4301, XP002289671 ISSN: 0006-2960 cited in the application
- LEHTO V-P: "EGF receptor: which way to go?" FEBS LETTERS, ELSEVIER SCIENCE PUBLISHERS, AMSTERDAM, NL, vol. 491, no. 1-2, 23 February 2001 (2001-02-23), pages 1-3, XP004257261 ISSN: 0014-5793
- ROSE KEITH ET AL: "Polyethyleneglycol-based chains of precise length for chemobodies" PEPTIDES FOR THE NEW MILLENNIUM KLUWER ACADEMIC PUBLISHERS, 3300 AA, DORDRECHT, NETHERLANDS; KLUWER ACADEMIC PUBLISHERS, 101 PHILLIP DRIVE, ASSINIPPI PARK, NORWELL, MA, 02061, USA, 2000, pages 47-49, XP009034147 & 16TH AMERICAN PEPTIDE SYMPOSIUM; MINNEAPOLIS, MI, USA; JUNE 26-JULY 01, 1999 ISSN: 0-7923-6445-7
- GARIEPY J ET AL: "Vectorial delivery of macromolecules into cells using peptide-based vehicles" TRENDS IN BIOTECHNOLOGY, ELSEVIER, AMSTERDAM,, GB, vol. 19, no. 1, 1 January 2001 (2001-01-01), pages 21-28, XP004227647 ISSN: 0167-7799
- NOMIZU MOTOYOSHI ET AL: "Multimeric forms of Tyr-Ile-Gly-Ser-Arg (YIGSR) peptide enhance the inhibition of tumor growth and metastasis" CANCER RESEARCH, vol. 53, no. 15, 1993, pages 3459-3461, XP001182805 ISSN: 0008-5472
- CARAGLIA M ET AL: "EGF activates an inducible survival response via the RASfwdarwErk-1/2 pathway to counteract interferon-alpha-mediated apoptosis in epidermoid cancer cells." CELL DEATH AND DIFFERENTIATION, vol. 10, no. 2, February 2003 (2003-02), pages 218-229, XP009034327 ISSN: 1350-9047
- MURAYAMA YOICHI: "High concentration of epidermal growth factor induces apoptosis in breast and esophageal cancer cells transplanted into nude mice" ONCOLOGY REPORTS, vol. 1, no. 6, 1994, pages 1055-1062, XP009034329
- GENNITY J ET AL: "SIGNAL PEPTIDE MUTANTS OF ESCHERICHIA COLI" JOURNAL OF BIOENERGETICS AND BIOMEMBRANES, PLENUM PUBLISHING, NEW YORK, NY, US, vol. 22, no. 3, June 1990 (1990-06), pages 233-269, XP009025834 ISSN: 0145-479X
- FATTAH ET AL: "Peptabody-EGF: A novel apoptosis inducer targeting ErbB1 receptor overexpressing cancer cells" INT. J. CANCER, vol. 119, 2006, pages 2455-2463,

## Description

### FIELD OF THE INVENTION

The present invention concerns multimeric molecules, which bind the epidermal growth factor receptor. More specifically, the present invention relates to an isolated and recombinant fusion peptabody binding to a member of the epidermal growth factor receptor, which can be used to treat diseases wherein expression of receptors is related to pathogenic phenotype. Examples of such diseases are: prostate cancer, bladder cancer, breast cancer, head and neck cancer, melanoma. Also disclosed are, nucleic acids encoding said isolated and recombinant fusion peptabody, kits and pharmaceutical compositions comprising said isolated and recombinant fusion peptabody as an active substance. Finally, a method for the manufacture of said peptabody, and its use for the preparation of a medicament for the treatment or prevention of cancer are provided.

### BACKGROUND OF THE INVENTION

Recent research has uncovered the important role played by the growth factor receptor tyrosine kinases in the etiology and development of human malignancies. These biological receptors are anchored by means of a transmembrane domain in the membranes of cells that express them. An extracellular domain binds to a growth factor. The binding of the growth factor to the extracellular domain results in a signal being transmitted to the intracellular kinase domain. The transduction of this signal is responsible for the proliferation and differentiation of the cells.

Members of the epidermal growth factor (EGF) receptor family are important growth factor receptor tyrosine kinases. The first member of the EGF receptor family of growth factors receptor to be discovered was a glycoprotein having an apparent molecular weight of approximately 165 kD. This glycoprotein, which was described by Mendelsohn et al. in U.S. patent 4,943,533, is known as the EGF receptor. The binding of EGF or transforming growth factor alpha (TGF-alpha) to the EGF receptor leads to cell growth.

Another member of the EGF receptor family of growth factor receptors is called erbB-2, also known as HER2, or p185 (Coussens et al., Science 230 (1985), 1132-1139; Yamamoto et al., Nature 319 (1986), 230-234). The receptor ErbB-2, which is expressed by the c-erbB-2 gene, is identical with the protein expressed by the human neu oncogene. The amino acid sequence of ErbB-2 is different from, but highly homologous to, that of the EGF receptor. ErbB-2 has been considered as an efficient target for cancer therapy. Herceptin, a monoclonal antibody raised against ErbB-2, is now being used in clinics with favourable results against metastatic breast cancer with ErbB-2 overexpression.

Other members of the EGF receptor family include erbB-3/HER-3 (Kraus et al., Proc. Natl. Acad. Sci. USA 86 (1989), 9193-9197; Plowman et al., Proc. Natl. Acad. Sci. USA 87 (1990), 4905-4909) and erbB-4/HER-4 (Plowman et al., Proc. Natl. Acad. Sci. USA 90 (1993), 1746-1750).

Many receptor tyrosine kinases are found in unusually high numbers on human tumors. For example, many tumors of epithelial origin express increased levels of EGF receptor on their cell membranes. Epidermal Growth Factor Receptor (EGFR) plays a central role in the cell life by controlling growth, proliferation, etc. Activation of the receptor occurs upon ligand binding and subsequent receptor dimerization (association of 2 receptors). Although EGFR is important in the maintenance of normal cellular function and survival, EGFR expression clearly appears to contribute to the growth and survival of tumor cells. Dysregulation of ErbB signalling can occur by diverse mechanisms including gene amplification and ErbB mutations that increase receptor transcription, translation or protein stability. Examples of tumors that express EGF receptors include glioblastomas as well as cancers of the lung, breast, head and neck, and bladder. The amplification and/or overexpression of the EGF receptors on the membranes of tumor cells is associated with a poor prognosis.

Various approaches have been developed to target ErbB family receptors including specific monoclonal antibodies (MAbs) specific against the extracellular region, inhibitors of the tyrosine kinase (TK) domain (required for receptor activation) and anti-sense therapy.

Antibodies, especially monoclonal antibodies, raised against tumor antigens have been investigated as potential anti-tumor agents. Such antibodies may inhibit the growth of tumors through a number of mechanisms. For example, antibodies may inhibit the growth of tumors immunologically through antibody-dependent cellular cytoxicity (ADCC) or complement-dependent cytoxicity (CDC).

Alternatively, antibodies may compete with the binding of a growth factor to its receptor, thereby inhibiting the growth of tumors that express the receptor. In another approach toxins are conjugated to antibodies raised against tumor antigens. The antibody portion directs the conjugate to the tumor, which is killed by the toxin portion.

Although the use of antibodies and antibody fragments as anti-tumor agents has shown promise, there are disadvantages that must be overcome. For example, monoclonal antibodies are produced in hybridomas. The use of hybridomas in commercial production is, however, less efficient than that of other expression systems such as bacteria. Moreover, the monoclonal antibodies are normally generated in animals other than humans. Such monoclonal antibodies are immunogenic in humans. This immunogenicity limits the effectiveness of the treatment of humans with such monoclonal antibodies.

The use of agents that block the activation of the EGFR TK was the second approach for the inhibition of EGFR pathway. Inhibitors compete with ATP for the binding of TK domain provoking a decrease of autophosphorylation. Some examples of inhibitors are ZD1839 from Astra Zeneca (Sweden), PKI-166 from Novartis (Switzerland), GSK572016 from Glaxo Smith Kline (UK). Although there are pharmacological and mechanistic differences between MAbs and inhibitors of TK, preclinical studies suggest they both inhibit cell proliferation and have additive or synergistic cytotoxicity with standard therapies.

Antisense technology has emerged as an exciting and promising strategy of cancer therapy but further developments are required to reach the clinical use. Few years ago, a promising new type of avidity molecule named "Peptabody" (Terskikh et al., 1997 "Peptabody: a new type of high avidity binding protein" Proc. Natl. Acad. Sci. USA 94(5): 1663-8) has been developed.

This peptabody molecule is also disclosed in WO 9818943 (Kajava et al.) as being an oligomer comprising more than 2 units, wherein each unit comprises a peptidic domain capable of oligomerizing and a domain capable of binding to an acceptor (ligand), wherein the oligomerizing domain is not an antibody or a functional antibody fragment from the constant region. Also described is the use and synthesis of this oligomer. The multimerization domain consists of a part of human cartilage oligomeric matrix polypeptide (COMP), which is fused to a hinge region or spacer (19 amino acids from human IgA). The binding domain of this peptabody, a short peptide ligand, is placed to the C-terminal part of the hinge.

This pentameric molecule has two main advantages in the targeting of effective receptors compared to monoclonal antibodies:
- By cooperative binding, its avidity for the target receptor can be dramatically increased.
- The crosslinking of the receptor may induce a strong biological effect on the target cells.

The concept of peptabody molecule allows a tight binding on cells expressing high level of targets whereas low density of target molecules is unfavourable to the peptabody binding.

Due to the short length of the monomer polypeptide chain, the oligomers can be synthesized chemically. The N-terminal position of the peptide ligands allows to synthesized first the core molecule (e.g. the pentamerization domain and a linker) and then split the sample and continue synthesis with different peptide sequences on the N-terminus. Thus oligomerization of short peptides bypass folding problems and overcomes expression difficulties previously experienced during oligomerization of relatively complex proteins such as single chain Fv fragments.

An attempt to synthesize a pentameric peptabody molecule reacting specifically with ErbB-2 receptor has been recently reported (Houimel et al., (1997) "Selection of peptides and synthesis of pentameric Peptabody molecules reacting specifically with ErbB-2 receptor" Int. J.Cancer 2001; 92: 748-755). Hexapeptides binding to ErbB-2 extracellular domain protein (ECD) have been selected from phage display libraries and used to construct a pentameric peptabody molecule as described above. However, no evidence of induction of apoptosis by the peptabody has been found. The observed inhibition of proliferation is probably due either to a partial agonist effect or to inhibition of receptor dimerization as described for anti-ErbB-2 MAb (Harwerth et al. "Monoclonal antibodies against the extracellular domain of ErbB-2 receptor function as partial ligand agonist" J. Biol. Chem. 1992; 267: 15160-7). Due to the relatively poor affinity of the selected hexapeptides to its ligand, it has been suggested to generate longer peptide molecules. However, because of its complexity the production of this type of recombinant fusion peptabody molecule has never been reported until now.

Recent studies demonstrated that tumors overexpressing the epidermal growth factor receptor (EGF-R, erbB-1) are associated with poor clinical outcome (Mendelsohn J (2002) "Targeting the epidermal growth factor receptor for cancer therapy" J. Clin. Oncol. 20:1 S-13S). There is, therefore, a continuing need for improved anti-tumor agents like "Peptabodies" that can be efficiently and inexpensively produced, have little or no immunogenicity in humans, and are capable of binding with an increased affinity to a member of the epidermal growth factor receptor, especially ErbB-1, that is expressed in high numbers on tumor cells. An object of the present invention is to provide such new anti-tumor agents that combine the advantageous features of the above techniques.

### SUMMARY OF THE INVENTION

These and other objects as will be apparent from the foregoing have been achieved by producing new recombinant fusion polypeptides which have a high potential as anti-tumor agents. In a first aspect, the object of the present invention is to provide an isolated and recombinant fusion peptabody, which binds to a member of the epidermal growth factor receptor useful in inhibiting the growth of certain tumor cells. Also disclosed are, nucleic acids encoding said isolated and recombinant fusion peptabody, kits and pharmaceutical compositions comprising said isolated and recombinant fusion peptabody as an active substance. Finally, a method for the manufacture of said isolated and recombinant fusion peptabody, and its use for the preparation of a medicament for the treatment or prevention of cancer are provided.

Other objects and advantages will become apparent to those skilled in the art from a review of the ensuing detailed description, which proceeds with reference to the following illustrative drawings, and the attendant claims.

### BRIEF DESCRIPTION OF THE FIGURES

Fig. 1: represents an illustration of the peptabody concept and the resulting avidity effect. The left part corresponds to a cell expressing a low level of receptors: peptabody binds one monomer without activation of EGFR pathway. The right part corresponds to a tumor cell expressing a high level of receptors: recruitment of EGFR leading to the cell death.
Fig. 2: shows the construction and production of Peptabodies anti-EGFR. The schematic representation of monomers of peptabody includes different portions: An Enhancer (sequence increasing the production in bacteria system), a histidine tail (6 x His), a hCOMP (49 a.a. of human oligomeric matrix polypeptide), a Hinge (19 a.a. of human IgA), and a hEGF (full length human epidermal growth factor). The amino acid sequences of an irrelevant peptabody and of a EGF peptabody are also represented.
Fig. 3: corresponds to a SDS PAGE and coomassie blue staining of monomer (m p-IRR, m p-EGF) and pentamer (P-IRR and P-EGF) of peptabody.
Fig. 4: represents the ELISA analysis of binding of Peptabodies and Mab-425 on human A431 cancer cell lines.
Fig. 5: represents a competition assay between Peptabody EGF and Mab-425 on human A431 cancer cell lines. Fixed concentration of Mab 425 (2.5 nM) was placed in presence of increasing concentrations of Peptabody EGF.
Fig. 6: shows a viability assay of epidermoïd cancer cells A431 in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 7: is a visualization of *in vitro* culture of epidermoïd cancer cells A431 in DMEM without serum after 2 days of treatment with peptabody EGF or peptabody Irrelevant p-IRR.
Fig. 8: shows a viability assay of human prostate cancer cells DU145 in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 9: is a visualization of *in vitro* culture of prostate cancer cells DU-145 in DMEM without serum after 2 days of treatment with peptabody EGF or peptabody Irrelevant p-IRR.
Fig. 10: shows a viability assay of human prostate cancer cells LNCaP in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 11: is a visualization of *in vitro* culture of prostate cancer cells LNCaP in DMEM without serum after 2 days of treatment with peptabody EGF or peptabody Irrelevant p-IRR.
Fig. 12: shows a viability assay of human prostate cancer cells PC-3 in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 13: is a visualization of *in vitro* culture of prostate cancer cells PC-3 in DMEM without serum after 2 days of treatment with peptabody EGF or peptabody Irrelevant p-IRR.
Fig. 14: shows a viability assay of human breast cancer cells MCF-7 in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 15: is a visualization of *in vitro* culture of breast cancer cells MCF-7 in DMEM with foetal calf serum after 2 days of treatment with peptabody EGF or peptabody Irrelevant p-IRR.
Fig. 16: shows a viability assay of human normal muscle cells in presence of different concentrations of Peptabodies EGF (p-EGF) or Irrelevant (p-IRR) at one, two and three days after treatment. The percentage (%) of inhibition is calculated by the ratio of viable cells in comparison to the control.
Fig. 17: is a visualization of *in vitro* culture of muscle cells in DMEM without serum after 2 days of treatment with peptabody EGF or peptabody Irrelevant p-IRR.
Fig. 18: shows viability assays of different human cancer cell lines (A431: human epidermoid cancer, MCF-7: human breast cancer, DU-145: human prostate cancer, UM-UC-3 and T24: human bladder cancer, Na8: human melanoma) in presence of 10 nM of Peptabodies EGF (p-EGF) or Irrelevant (p-IRR) at one, three, six, and 24 hours after treatment. The percentage (%) of inhibition is calculated by the ratio of viable cells in comparison to the control.
Fig. 19: corresponds to a FACS Annexin V staining protocol to measure the apoptotic effect of peptabody EGF or peptabody Irrelevant pIRR on human A431 epidermoid cancer cells. Epidermoid A-431 cancer cells were treated for 0 min. (T₀), 30 min (T₃₀), 60 min (T₆₀), 180 min (T₁₈₀) or 360 min (T₃₆₀) with 10 nM of p-EGF and with 10 nM of p-IRR, respectively. Changes in Annexin V-FITC binding were analysed by FACS. Fig. 19A: Comparison of p-EGF (thick line) and p-IRR (thin line), induced apoptosis. Fig. 19B: Time course of peptabody induced apoptosis.
Fig. 20: corresponds to a SDS-PAGE analysis of Peptabodies under non- or reducing conditions. MDP01: peptabody EGF, MDP03: peptabody GBP, MDP00: irrelevant peptabody.
Fig. 21: represents a cytotoxic assay on cancer cells using MTT method. Cancer cells were incubated with Peptabodies, or monoclonal antibodies specific to EGFR at a concentration of 2 µg/ml .
Fig. 22: shows the DNA sequence and the protein sequence of an Irrelevant Peptabody: MDP00. In Italic is the start codon ATG (methionine) and stop codon TAA (*), in bold is the enhancer peptide, underlined is the His tag, in normal characters is the human COMP and in Italic and bold is the Hinge region (human).

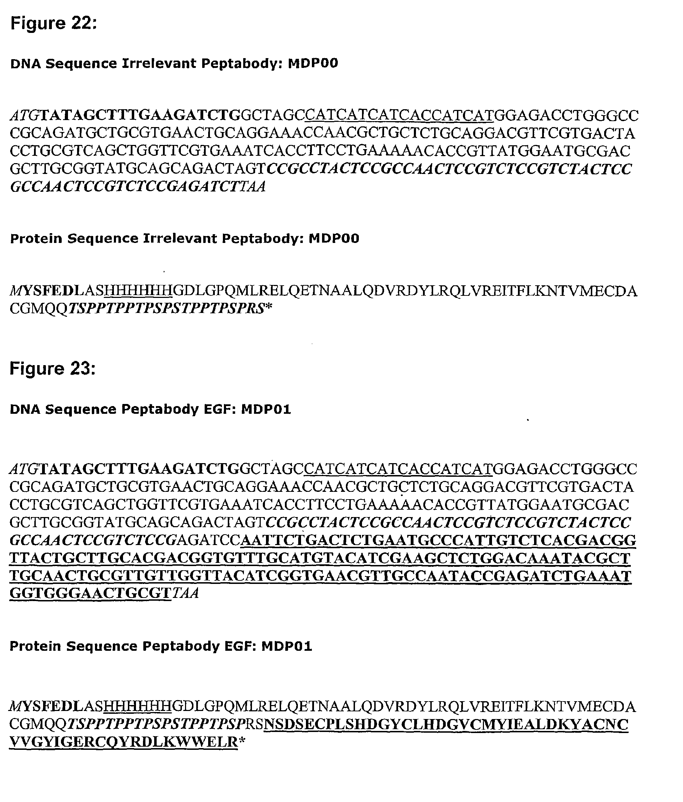

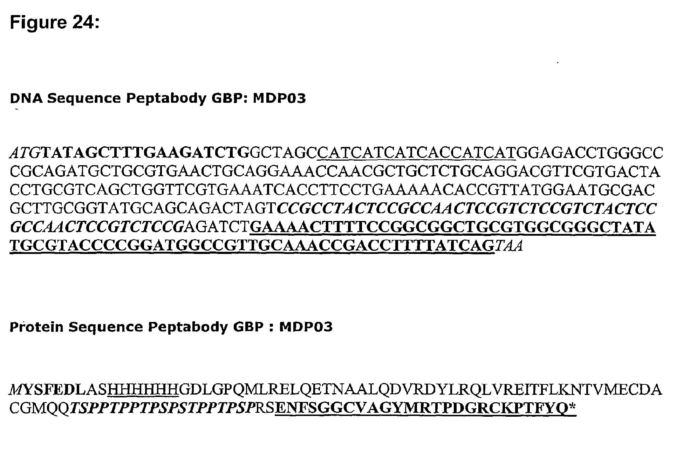

Fig. 23: shows the DNA sequence and the protein sequence of a Peptabody EGF: MDP01. In Italic is the start codon ATG (methionine) and stop codon TAA (*), in bold is the enhancer peptide, underlined is the His tag, in normal characters is the human COMP, in Italic and bold is the Hinge region (human) and in bold and underlined is the epidermal growth factor (EGF). Fig. 24: shows the DNA sequence and the protein sequence of a Peptabody GBP: MDP03. In Italic is the start codon ATG (methionine) and stop codon TAA (*), in bold is the enhancer peptide, underlined is the His tag, in normal characters is the human COMP, in Italic and bold is the Hinge region (human) and in bold and underlined is the growth binding peptide (GBP).
Fig. 25: relates to the production of decabodies fused to different enhancers. A Coomassie blue staining of SDS PAGE of decabody production using a bacteria system is represented. A) corresponds to the insoluble fraction, B) corresponds to the soluble fraction.
Fig. 26: relates to the production of Peptabodies fused to different enhancers. A Western blot analysis of peptabody production using a bacteria system is represented. The detection is performed with anti-His antibody against an urea bacteria extract.
Fig. 27: represents the plasmid map pQE-09.

### DETAILED DESCRIPTION OF THE INVENTION

As used herein, the following definitions are supplied in order to facilitate the understanding of the present invention.

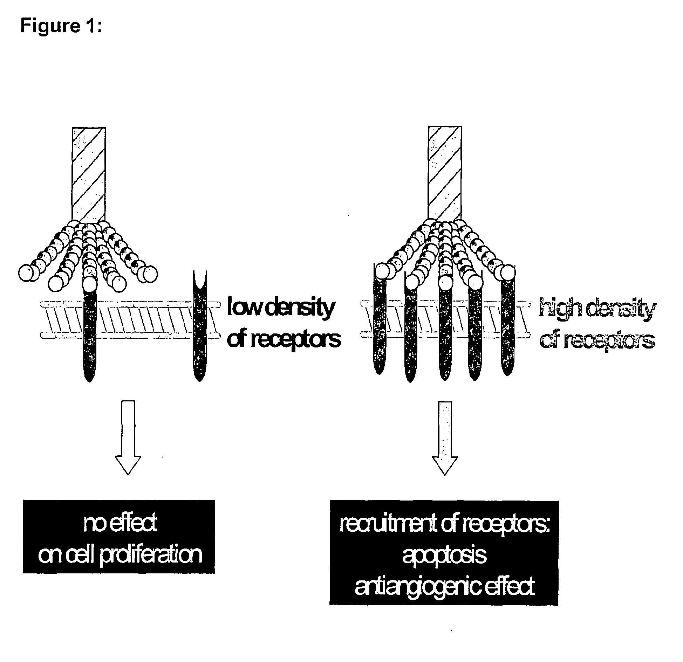

A "Peptabody" as disclosed in WO 9818943 (Kajava et al.) is a high avidity molecule which uses the multimerization concept for inducing aberrant cell signals. The multimerization domain consists of a part of human cartilage oligomeric matrix protein (COMP), which is fused to a hinge region or spacer (preferably containing 19 amino acids from human IgA) and a domain (binding domain) capable of binding to an acceptor (ligand). The peptabody has two main advantages toward monoclonal antibodies for the targeting of effective receptors: 1) by cooperative binding, its avidity for the target receptor can be dramatically increased; and 2) the tight crosslinking of receptors may cause a strong biological effect on the targeted cells inducing an arrest of cell growth and/or apoptosis (see Fig. 1). The concept of peptabody molecule allows a tight binding on cells expressing high level of targets whereas low density of target molecules is unfavourable to the peptabody binding. "Decabodies" are constructed on the same principle with the difference that they possess ten arms and consequently ten binding domains.

A member of the "ErbB receptor" is a receptor protein tyrosine kinase which belongs to the ErbB receptor family and includes EGFR, ErbB3 and ErbB4 receptors and other members of this family to be identified in the future. ErbB2 receptor is not comprised in this definition and thus is not a target of the isolated and recombinant fusion peptabody according to the present invention. The ErbB receptor will generally comprise an extracellular domain, which may bind an ErbB ligand; a lipophilic transmembrane domain; a conserved intracellular tyrosine kinase domain; and a carboxyl-terminal signalling domain harbouring several tyrosine residues which can be phosphorylated. The ErbB receptor may be a "native sequence" ErbB receptor or an "amino acid sequence variant" thereof. Preferably the ErbB receptor is native sequence human ErbB receptor.

The terms "ErbB1 ", "epidermal growth factor receptor" and "EGFR" are used interchangeably herein and refer to EGFR as disclosed, for example, in Carpenter et al. Ann. Rev. Biochem. 56:881-914 (1987), including naturally occurring isoforms or mutant forms thereof (e.g. a deletion mutant EGFR as in Humphrey et al. PNAS (USA) 87:4207-4211 (1990)). The term erbB1 refers to the gene encoding the EGFR protein product.

The expressions "ErbB2" and "HER2" are used interchangeably herein and refer to human HER2 protein described, for example, in Semba et al., PNAS (USA) 82:6497-6501 (1985) and Yamamoto et al. Nature 319:230-234 (1986) (Genebank accession number X03363). The term "erbB2" refers to the gene encoding human ErbB2 and "neu" refers to the gene encoding rat p185. As defined above, ErbB2 receptor is not a target of the isolated and recombinant fusion peptabody of the present invention and therefore is excluded from the scope of this invention.

"ErbB3" and "HER3" refer to the receptor polypeptide as disclosed, for example, in U.S. Pat. Nos. 5,183,884 and 5,480,968 as well as Kraus et al. PNAS (USA) 86:9193-9197 (1989).

The terms "ErbB4" and "HER4" herein refer to the receptor polypeptide as disclosed, for example, in EP 599 274; Plowman et al., Proc. Natl. Acad. Sci. USA, 90:1746-1750 (1993); and Plowman et al., Nature, 366:473-475 (1993), including isoforms and mutant forms thereof, e.g., as disclosed in WO99/19488.

By "ErbB ligand" is meant a polypeptide which binds to and/or activates an ErbB receptor. The EGF family of ligands can be divided in three groups based on their ability to bind and activate distinct ErbB receptor homo and heterodimers (Lopez-Torrejon et al., 2002 "Human betacellulin structure modelled from other members of EGF family" J. Mol. Model. 8:131-44). The first group is composed of EGF, amphiregulin and transforming growth factor alpha TGFα, and binds directly with EGFR. The second group, constituted by heregulins, requires for binding the formation of heterodimers with ErbB-3 or -4. The third group are bispecific ligands because of their binding on either EGFR or ErbB-4. Betacellulin, heparin binding - epidermal growth factor, and epiregulin are from this group.

The ErbB ligands of particular interest herein are: a native sequence human ErbB ligand such as epidermal growth factor (EGF) (Savage et al., J. Biol. Chem. 247:7612-7621 (1972)); transforming growth factor alpha (TGF-[alpha]) (Marquardt et al., Science 223:1079-1082 (1984)); amphiregulin also known as schwanoma or keratinocyte autocrine growth factor (Shoyab et al. Science 243:1074-1076(1989); Kimura et al. Nature 348:257-260(1990); and Cook et al. Mol. Cell. Biol. 11:2547-2557 (1991)); betacellulin (Shing et al., Science 259:1604-1607 (1993); and Sasada et al. Biochem. Biophys. Res. Commun. 190:1173 (1993)); heparin-binding epidermal growth factor (HB-EGF) (Higashiyama et al., Science 251:936-939 (1991 )); epiregulin (Toyoda et al., J. Biol. Chem. 270:7495-7500 (1995); and Komurasaki et al. Oncogene 15:2841-2848 (1997)); a heregulin (see below); neuregulin-2 (NRG-2) (Carraway et al., Nature 387:512-516 (1997)); neuregulin-3 (NRG-3) (Zhang et al., Proc. Natl. Acad. Sci. 94:9562-9567 (1997)); neuregulin-4 (NRG-4) (Harari et al. Oncogene 18:2681-89(1999)) or cripto (CR-1) (Kannan et al. J. Biol. Chem. 272(6):3330-3335 (1997)). ErbB ligands which bind EGFR include EGF, TGF-[alpha], amphiregulin, betacellulin, HB-EGF and epiregulin. ErbB ligands that bind ErbB3 include heregulins. ErbB ligands capable of binding ErbB4 include betacellulin, epiregulin, HB-EGF, NRG-2, NRG-3, NRG-4 and heregulins. Viral EGF-like ligands such as VGF (vaccinia growth factor), SFGF (shope fibroma growth factor) or MGF (Myxoma Growth Factor) are also encompassed by the present invention.

A "native sequence" polypeptide is one which has the same amino acid sequence as a polypeptide (e.g., ErbB receptor or ErbB ligand) derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

"Fragments" refer to sequences sharing at least 50% amino acids in length with the respective sequence of the polypeptide capable of binding to the epidermal growth factor receptor. These sequences can be used as long as they exhibit the same properties as the native sequence from which they derive. Preferably these sequences share at least 70%, more preferably more than 80%, in particular more than 90% amino acids in length with the respective sequence of polypeptide capable of binding to the EGFR.

Also disclosed are "variants" of the aforementioned sequences capable of binding to the epidermal growth factor receptor. The terms "amino acid sequence variant" or "variants" refer to polypeptides having amino acid sequences that differ to some extent from a native sequence polypeptide, that is amino acid sequences that vary from the native sequence by conservative amino acid substitutions, whereby one or more amino acids are substituted by another with same characteristics. Ordinarily, amino acid sequence variants will possess at least about 70% homology with at least one receptor binding domain of a native ErbB ligand or with at least one ligand binding domain of a native ErbB receptor, and preferably, they will be at least about 80%, more preferably at least about 90% homologous with such receptor or ligand binding domains. The amino acid sequence variants possess substitutions, deletions, and/or insertions at certain positions within the amino acid sequence of the native amino acid sequence. Conservative amino acid substitutions are herein defined as exchanges for example within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gln
IV. Large, aromatic residues: Phe, Tyr, Trp
V. Large, aliphatic, nonpolar residues: Met, Leu, Ile, Val, Cys.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2", authored by Genentech, Inc., which was filed with user documentation in the United States Copyright Office, Washington, D.C. 20559, on Dec. 10, 1991.

An "enhancer" sequence of the peptabody is a peptide sequence placed at the N-terminal part of the isolated and recombinant fusion peptabody or decabody to increase significantly the production of peptabody or decabody in prokaryotic or Eukaryotic system.

"Promoter" as used herein refers to a nucleic acid sequence that regulates expression of a gene. A "promoter sequence" is a DNA regulatory region capable of binding RNA polymerase in a cell and initiating transcription of a downstream (3'direction) coding sequence. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine Dalgarno sequences in addition to the-10 and-35 consensus sequences.

A nucleic acid sequence is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter is operably linked to a coding sequence if it affects the transcription of the sequence; or a ribosome binding site is operably linked to a coding sequence if it is positioned so as to facilitate translation. Linking is accomplished by ligation at convenient restriction sites. If such sites do not exist, the synthetic oligonucleotide adaptors or linkers are used in accordance with conventional practice.

The term "isolated" and "purified" refers to the state in which the recombinant fusion peptabody of the invention, or nucleic acid encoding such recombinant fusion peptabody will be, in accordance with the present invention. Peptabody molecules and nucleic acid will be free or substantially free of material with which they are naturally associated such as other polypeptides or nucleic acids with which they are found in their natural environment, or the environment in which they are prepared (e. g. cell culture) when such preparation is by recombinant DNA technology practised in vitro or in vivo.

The terms "introducing a purified DNA into a eukaryotic or prokaryotic host cell" or "transfection" denote any process wherein an extracellular DNA, with or without accompanying material, enters a host cell. The term "cell transfected" or "transfected cell" means the cell into which the extracellular DNA has been introduced and thus harbours the extracellular DNA. The DNA might be introduced into the cell so that the nucleic acid is replicable either as a chromosomal integrant or as an extra chromosomal element.

The terms "host cell" and "recombinant host cell" are used interchangeably herein to indicate a eukaryotic or prokaryotic cell into which one or more DNA or vectors of the invention have been introduced. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

"Eukaryotic cell" refers to any mammalian or non-mammalian cell from a eukaryotic organism. By way of non-limiting example, any eukaryotic cell that is capable of being maintained under cell culture conditions and subsequently transfected would be included in this invention. Especially preferable cell types include, e. g., stem cells, Chinese hamster ovary cells (CHO), COS, BHK21, NIH3T3, HeLa, C2C12, cancer cells, and primary differentiated or undifferentiated cells. Other suitable host cells are known to those skilled in the art.

A Peptabody which induces "apoptosis" or "necrosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses the ErbB receptor. Preferably the cell is a tumor cell, e.g. a breast, prostate, ovarian, stomach, endometrial, salivary gland, lung, kidney, colon, thyroid, pancreatic or bladder cell. In vitro, the cell may be a SK-BR-3, BT474, Calu 3 cell, MDA-MB-453, MDA-MB-361 or SKOV3 cell. Various methods are available for evaluating the cellular events associated with apoptosis. For example, phosphatidyl serine (PS) translocation can be measured by annex in binding; DNA fragmentation can be evaluated through DNA laddering; and nuclear/chromatin condensation along with DNA fragmentation can be evaluated by any increase in hypodiploid cells.

"Treatment" refers to both therapeutic treatment and prophylactic or preventative measures. Those in need of treatment include those already with the disorder as well as those in which the disorder is to be prevented. Hence, the mammal to be treated herein may have been diagnosed as having the disorder or may be predisposed or susceptible to the disorder.

"Mammal" for purposes of treatment refers to any animal classified as a mammal, including humans, domestic and farm animals, and zoo, sports, or pet animals, such as dogs, horses, cats, cows, monkeys etc. Preferably, the mammal is human.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. The phrase "therapeutically effective amount" is used herein to mean an amount sufficient to prevent, and preferably reduce by at least about 30 percent, preferably by at least 50 percent, preferably by at least 70 percent, preferably by at least 80 percent, preferably by at least 90%, a clinically significant change in the growth or progression or mitotic activity of a target cellular mass, group of cancer cells or tumor, or other feature of pathology.

The terms "cancer" and "cancerous" refer to or describe the physiological condition in mammals that is typically characterized by unregulated cell growth. Examples of cancer include, but are not limited to, carcinoma, lymphoma, blastoma, sarcoma (including liposarcoma), neuroendocrine tumors, mesothelioma, schwanoma, meningioma, adenocarcinoma, melanoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophagael cancer, tumors of the biliary tract, as well as head and neck cancer.

An "ErbB-expressing cancer" is one comprising cells which have ErbB protein present at their cell surface.

A cancer "characterized by excessive activation" of an ErbB receptor is one in which the extent of ErbB receptor activation in cancer cells significantly exceeds the level of activation of that receptor in non-cancerous cells of the same tissue type. Such excessive activation may result from overexpression of the ErbB receptor and/or greater than normal levels of an ErbB ligand available for activating the ErbB receptor in the cancer cells. Such excessive activation may cause and/or be caused by the malignant state of a cancer cell. In some embodiments, the cancer will be subjected to a diagnostic or prognostic assay to determine whether amplification and/or overexpression of an ErbB receptor is occurring which results in such excessive activation of the ErbB receptor. Alternatively, or additionally, the cancer may be subjected to a diagnostic or prognostic assay to determine whether amplification and/or overexpression an ErbB ligand is occurring in the cancer which attributes to excessive activation of the receptor.

A cancer which "overexpresses" an ErbB receptor is one which has significantly higher levels of an ErbB receptor, such as ErbB1, at the cell surface thereof, compared to a noncancerous cell of the same tissue type. Such overexpression may be caused by gene amplification or by increased transcription or translation. ErbB receptor overexpression may be determined in a diagnostic or prognostic assay by evaluating increased levels of the ErbB protein present on the surface of a cell (e.g. via an immunohistochemistry assay; IHC).

The term "comprise" is generally used in the sense of include, that is to say permitting the presence of one or more features or components.

The phrase "pharmaceutically acceptable" refers to molecular entities and compositions that are physiologically tolerable and do not typically produce an allergic or similar untoward reaction, such as gastric upset, dizziness and the like, when administered to a human.

The term "humanized" forms of non-human (e.g., rodent) portions of the Peptabody, especially the humanized cartilage oligomer matrix polypeptide and the portion of a hinge region of an immunoglobulin polypeptide, refer to chimeric parts thereof that contain minimal sequence derived from non human immunoglobulin.

By "chimeric" part, is intended an amino acid sequence that may include a functional portion of the sequence and that will be obtained by molecular biology methods known by those skilled in the art.

"Chimeric protein" refers to a protein comprising two or more polypeptides, which are from different origins, i.e. which do not occur together in the nature.

The polypeptide according to the present invention is an isolated and recombinant fusion peptabody which can be encoded by a single DNA sequence. The isolated and recombinant fusion peptabody is able to bind to a member of the epidermal growth factor receptor which is ErbB1, ErbB3 or ErbB4 and preferably ErbB1. The isolated and recombinant fusion peptabody of the present invention comprises at least:
(a) a portion of a humanized or human cartilage oligomer matrix polypeptide;
(b) a portion of a hinge and
(c) an epidermal growth factor receptor ligand comprising at least a motif having a three-dimensional structure. The isolated and recombinant fusion peptabody is capable of inducing cellular death (apoptosis and/or necrosis) in a cell that expresses epidermal growth factor receptors, preferably a cancer cell and more preferably an ErbB-expressing cancer cell. ErbB-expressing cancer cells are preferably cells characterized by excessive activation of an ErbB receptor or cells which "overexpresses" an ErbB receptor.

A "three-dimensional structure" or tertiary structure is the overall 3D structure of a protein or in other words the conformation into which a protein or a polypeptide folds. A protein molecule is made of a long chain of amino acid sequences that fold into a complex three-dimensional structure. It is often the geometrical shapes that determine the protein functions.

The epidermal growth factor receptor ligand of the present invention, comprises a long amino-acid sequence, preferably, the EGFR ligand is a complex polypeptide and more preferably a protein. It will be understood that the epidermal growth factor receptor ligand is preferably present in its full-length sequences. Table 1, shows that the minimal amino-acid sequence necessary for binding the EGFR with a strong affinity and activity comprises a sequence of at least 10 amino-acids, preferably 20 more preferably more than 20 amino-acids. In the case of an EGF polypeptide ligand, it has been shown that the minimal EGF region required for efficient EGFR activation is a structural motif involving three site loops resulting of the folding of the protein.

EGF and all other members of this growth factor family have a characteristic spacing of six conserved cysteine residues, arranged in three disulfide bridges. In combination with two conserved glycine residues, this provides the three-dimensional scaffold that is required for proper ErbB receptor binding (Campbell et al., 1990. Biochem. Pharmacol. 40, 35-40). On the basis of their cysteine spacing, these molecules can be divided into three looped regions, designated the A-loop (Cys6-Cys20), B-loop (Cys14-Cys31), and C-loop (Cys33-Cys42), in addition to a linear N-terminal region, a linear C-terminal region, and a single amino acid hinge region between the fourth and the fifth cysteine (Stortelers et al., 2002. Biochemistry, 41 (13), 4292-4301). High affinity binding requires a proper three-dimensional structure of the ligand in combination with specific residues in the EGF receptor binding domain.

The isolated and recombinant fusion peptabody recognizes an EGFR molecule on the surface of a cell, preferably a eukaryotic cell, more preferably, a vertebrate cell and, most preferably, a mammal cell, e.g. a human cell. The binding is specific for a defined structure on the cell surface. Binding also takes place with high affinity, preferably with a binding constant between 5-10 nM and preferably of 9 nM.

Preferably, the isolated and recombinant fusion peptabody of the present invention is fully human or humanized. In particular, the cartilage oligomer matrix polypeptide and the portion of a hinge are humanized or fully human. The peptidic domain capable of oligomerizing (COMP, i.e. the portion of a humanized or human cartilage oligomer matrix polypeptide) and the domain capable of binding to a member of the epidermal growth factor receptor are connected via a spacer (hinge region) to provide, for example, a greater flexibility of the binding domain. For example, a proline-rich sequence of a spacer is supposed to prevent formation of secondary structure elements and as a consequence a fixed 3-D structure. Moreover, a spacer region like that is generally supposed to be quite rigid due to the conformational constrains of the proline residues, bringing a beneficial effect for the cooperativity of the multivalent binding. Accordingly, in a preferred embodiment of the invention, the portion of a humanized or human cartilage oligomer matrix polypeptide capable of oligomerizing and the epidermal growth factor receptor ligand capable of binding to a member of the epidermal growth factor receptor are connected via a portion of a hinge region of an immunoglobulin polypeptide (spacer), that comprises preferably a proline-rich region. More preferably, the portion of a hinge is a region of an immunoglobulin polypeptide.

The portion of a humanized or human cartilage oligomer matrix polypeptide (COMP) is a peptidic domain capable of oligomerizing and of self-assembling whereby it can spontaneously pentamerize. A preferred embodiment of the inventive oligomer represents a self-assembling molecule able to oligomerize in vivo and/or in vitro.

In a preferred embodiment, the isolated and recombinant fusion peptabody is multimeric i.e. dimers or trimers of peptabody molecules.

The construction of the isolated and recombinant fusion peptabody of the present invention is different from the prior art in that: the portion of the hinge preferably a region of an immunoglobulin polypeptide is located at the C terminus of the portion of the humanized cartilage oligomer matrix polypeptide, the epidermal growth factor receptor ligand is located at the C terminus of the hinge region and the enhancer sequence is located at the N terminus of the portion of the humanized cartilage oligomer matrix polypeptide. This difference leads to an improved efficacy and a better production rate.

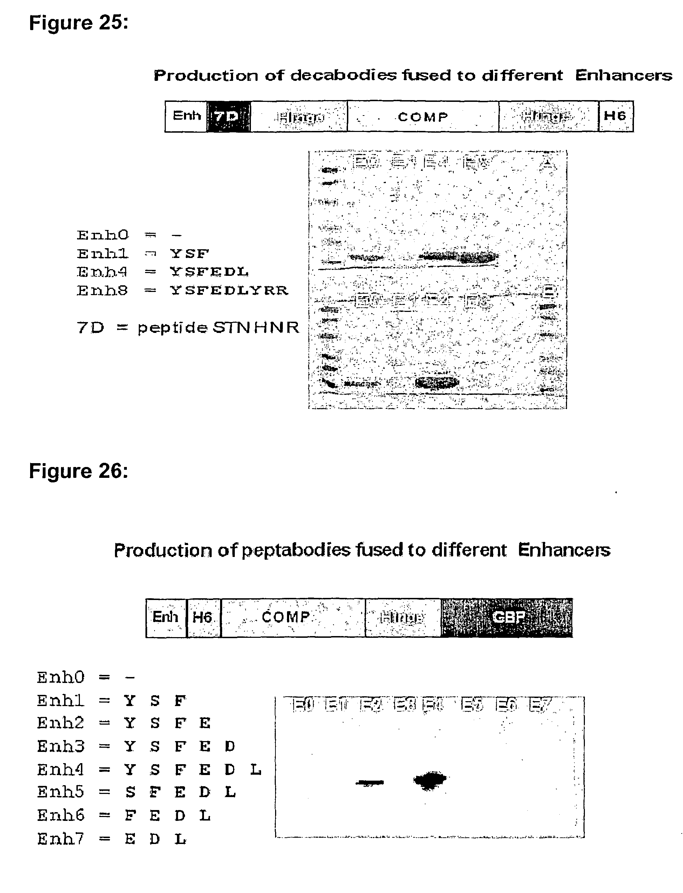

The isolated and recombinant fusion peptabody of the present invention further comprises an enhancer sequence. The enhancer sequence is selected from the group comprising: YSFE, YSFEDL, and YSFEDLYRR, a fragment thereof, a molecular chimera thereof and variants thereof. It has been shown that the presence of an enhancer sequence at the N terminus and preferably the above selected enhancers leads to a production rate which is 20 to 100 fold higher (see Fig. 25 and 26).

The epidermal growth factor receptor ligand is selected among the group of:
(a) an epidermal growth factor polypeptide or fragments thereof,
(b) a growth blocking peptide or fragments thereof,
(c) a TGF alpha polypeptide or fragments thereof,
(d) an amphiregulin polypeptide or fragments thereof,
(e) a heparin-binding epidermal growth factor-like polypeptide or fragments thereof,
(f) a betacellulin polypeptide or fragments thereof.

It will be understood that chimeric parts thereof are also envisaged. Fragments of epidermal growth factor receptor ligands can be used as long as they exhibit the same properties as the native sequence from which they derive.

**Table 1 shows the various EGF receptor ligands which have been fused to the isolated and recombinant fusion peptabody of the present invention.**

| **Name** | | **Sequence** | | **Origin** |
|---|---|---|---|---|
| Plasmocyte Spreading Peptide 1 | Psi PSP1 | ENFNGGCLAGYMRTADGRCKPTF | 23 a.a. | *Pseudoplusia includens* |
| Sp1 Growth Blocking Peptide | Sp1 GBP | ENFSGGCVAGYMRTPDGRCKPTFYQ | 25 a.a. | *Spodoptera litura* |
| Mab Growth Blocking Peptide | Mab GBP | ENFAAGCATGYQRTADGRCKPTF | 23 a.a. | *Mamestra brassicae* |
| Spe Paralytic Peptide 1 | Spe PP1 | ENFAGGCATGYLRTADGRCKPTF | 23 a.a. | *Spodoptera eridania* |
| Mas Paralytic Peptide 1 | Mas PP1 | ENFAGGCAAGYLRTADGRCKPTF | 23 a.a. | *Manduca sexta* |
| Mas Paralytic Peptide 2 | Mas PP2 | ENFAGGCATGFLRTADGRCKPTF | 23 a.a. | *Manduca sexta* |
| Hev Paralytic Peptide 1 | Hev PP1 | ENFSGGCIPGYMRTADGRCKPTY | 23 a.a. | *Heliothis virescens* |
| Hev Paralytic Peptide 2 | Hev PP2 | ENFAGGCIPGYMRTADGRCKPTY | 23 a.a. | *Heliothis virescens* |
| Trn Paralytic Peptide 1 | Trn PP1 | ENFSGGCLAGYMRTADGRCKPTFG | 24 a.a. | *Trichoplusia ni* |
| Trn Paralytic Peptide 2 | Trn PP2 | ENFSGGCLAGYMRTADGRCKPTF | 23 a.a. | *Trichoplusia ni* |
| Any Paralytic Peptide | Any PP | ENFAGGCATGFMRTADGRCKPTF | 23 a.a. | *Antheraea yamamai* |
| Spe Cardioactive Peptide | Spe CAP | ENFAVGCTPGYQRTADGRCKPTF | 23 a.a. | *Spodoptera eridania* |
| Spe Paralytic Peptide 1 | Spe PP1 | ENFAGGCTPGYQRTADGRCKATF | 23 a.a. | *Spodoptera eridania* |
| Spe Paralytic Peptide 2 | Spe PP2 | ENFAGGCTPGYQRTADGRCKPTF | 23 a.a. | *Spodoptera eridania* |
| Spe Paralytic Peptide 3 | Spe PP3 | ENFVGGCTPGYQRTADGRCKPTF | 23 a.a. | *Spodoptera eridania* |
| Transforming Growth Factor α | TGFα | | 50a.a | *Human* |
| Amphiregulin | AR | | 84 a.a. | *Human* |
| Heparin-binding EGF-like growth factor | HB-EGF | | 86 a.a. | *Human* |
| Betacellulin | HBT C | | 80 a.a. | *Human* |
| Betacellulin (C-terminal part) | HBT C | | 50 a.a. | *Human* |

### Table 1

For a better separation, the isolated and recombinant fusion peptabody of the invention further comprises a polyhistidine tag sequence. The separation may be carried out by affinity chromatography or any other convenient techniques known to the skilled in the art. For affinity chromatography purification, any antibody which specifically binds to the isolated and recombinant fusion peptabody or to the polyhistidine tag sequence may be used. Other affinity molecules such as Ni²⁺-nitrilotriacetic linked to agarose beads and which bind specifically to the polyhistidine tag sequence are also envisioned in the present invention.

Furthermore, the N-terminus of the isolated and recombinant fusion peptabody is preferably modified by the addition of at least one further functional domain or effector region, like a marker, an enzymatic or a cytotoxic region, or a region that adds additional binding properties, for example to metal atoms or other structural compounds, that can be used, e.g., in affinity chromatography. The effector region may be attached to the isolated and recombinant fusion peptabody of the invention using conventional chemistry known in the art.

Examples of effector regions are detectable molecules or detection moiety for diagnostic purposes, such as enzymes or peptides having a particular binding property, e.g. streptavidin or horseradish peroxidase. Detection moiety further includes chemical moieties such as biotin which may be detected via binding to a specific cognate detectable moiety, e. g. labelled avidin.

Detection moiety also includes fluorescent labels and labels used conventionally in the art for MRI-CT imagine. A number of fluorescent materials are known and can be utilized as labels. These include, for example, fluorescein, rhodamine, auramine, Texas Red, AMCA blue and Lucifer Yellow.

The isolated and recombinant fusion peptabody may carry a radioactive label as the detection moiety, such as the isotopes 3H, 14C, 32P, 35S, 36Cl, 51 Cr, 57Co, 58Co, 59Fe, 90Y, 121I, 124I, 125I, 131I, 111In, 211At, 198Au, 67Cu, 225Ac, 213bu, 99Tc and 186Re. When radioactive labels are used, known currently available counting procedures may be utilized to identify and quantitate the specific binding members. In the instance where the label is an enzyme, detection may be accomplished by any of the presently utilized colorimetric, spectrophotometric, fluorospectrophotometric, amperometric or gasometric techniques known in the art.

The radiolabelled isolated and recombinant fusion peptabody, are useful in *in vitro* diagnostics techniques and in *in vivo* radioimaging techniques. In a further aspect of the invention, radiolabelled isolated and recombinant fusion peptabody, are useful in radioimmunotherapy, particularly for cancer therapy. In a still further aspect, the radiolabelled isolated and recombinant fusion peptabody, are useful in radioimmuno-guided surgery techniques, wherein they can identify and indicate the presence and/or location of cancer cells, precancerous cells, tumor cells, and hyperproliferative cells, prior to, during or following surgery to remove such cells.

In the instance of in vivo imaging, the isolated and recombinant fusion peptabody of the present invention may be conjugated to an imaging agent rather than a radioisotope(s), including but not limited to a magnetic resonance image enhancing agent, wherein for instance an isolated and recombinant fusion peptabody molecule is loaded with a large number of paramagnetic ions through chelating groups. Examples of chelating groups include EDTA, porphyrins, polyamines crown ethers and polyoximes.

Examples of paramagnetic ions include gadolinium, iron, manganese, rhenium, europium, lanthanium, holmium and erbium.

Preferably, the effector region comprises a cytotoxin, i.e. a polypeptide which can inhibit cell growth or cause the destruction of a cell. The cytotoxin can be a bacterial cytotoxin, such as diphtheria toxin derived from Corynebacterium diphtheriae, exotoxin A derived from Pseudomonas aeruginosa, anthrax toxins derived from Bacillus anthracis, Shigatoxin derived from Shigella, Shiga-like toxins derived from Escherichia coli, or pertussis toxins derived from Bordatella pertussis or a toxic region thereof. Preferably, a bacterial cytotoxin is exotoxin A from Pseudomonas aeruginosa (ATCC 25313-25363) or a toxic region thereof, e.g. the domains II, Ib and/or III.

On the other hand, the cytotoxin can also be derived from plants. Examples of plant cytotoxins are ricin (Lamb et al., Eur.J.Biochem. 148, 265-270 (1985)), abrin (Wood et al., Eur.J.Biochem. 198, 723-732 (1991)), a ribozyme inactivating protein, such as saporin (Benatti et al., Eur.J.Biochem. 183, 465-470 (1989)), pokeweed antiviral protein (Kataoka et al., Plant Mol.Biol.20, 879-886 (1992)), gelonin (Nolan et al., Gene 134, 223-227 (1993)) or trichosanthin (Shaw et al., Gene 97, 267-272 (1991)).

In a further preferred embodiment the effector region comprises a ribonuclease, particularly a ribonuclease derived from a mammal, e.g. bovine pancreatic RNase A (Carsana et al., Nucleic Acids Res. 16, 5491-5502 (1988)), human angiogenin (Kurachi et al., Biochemistry 24, 5494-5499 (1985)) or human eosinophil-derived neurotoxin (Rosenberg et al., Proc.Natl.Acad.Sci. USA 86, 4460-4464 (1989)). Most preferably, the ribonuclease is derived from a human.

Other cytotoxic drugs may be used such as 5-fluorouracil or ricin and enzymes such as bacterial carboxypeptidase or nitroreductase, which are capable of converting prodrugs into active drugs at the site of a tumor.

Isolated and recombinant fusion Peptabodies may be administered to a patient in need of treatment via any suitable route, usually by injection into the bloodstream or CSF, or directly into the site of the tumor or close to this site. The precise dose will depend upon a number of factors, including whether the Peptabody is for diagnosis or for treatment, the size and location of the tumor, the precise nature of the Peptabody, and the nature of the detectable or functional label attached to the isolated and recombinant fusion peptabody. Where a radionuclia is used for therapy, a suitable maximum single dose is about 45 mCi/m2, to a maximum of about 250 mCi/m2. Preferable dosage is in the range of 15 to 40 mCi, with a further preferred dosage range of 20 to 30 mCi, or 10 to 30 mCi.

Another subject matter of the present invention is an isolated and purified DNA molecule encoding the isolated and recombinant fusion Peptabody as defined above and a vector comprising at least one copy of this isolated and purified DNA molecule.

DNA which can be used herein is any polydeoxynuclotide, including, e.g. double-stranded DNA, single-stranded DNA, double-stranded DNA wherein one or both strands are composed of two or more fragments, double-stranded DNA wherein one or both strands have an uninterrupted phosphodiester backbone, DNA containing one or more single-stranded portion(s) and one or more double-stranded portion(s), double-stranded DNA wherein the DNA strands are fully complementary, double-stranded DNA wherein the DNA strands are only partially complementary, circular DNA, covalently-closed DNA, linear DNA, covalently cross-linked DNA, cDNA, chemically- synthesized DNA, semi-synthetic DNA, biosynthetic DNA, naturally-isolated DNA, enzyme-digested DNA, sheared DNA, labeled DNA, such as radiolabeled DNA and fluorochrome-labeled DNA, DNA containing one or more non-naturally occurring species of nucleic acid. DNA sequences that encode the isolated and recombinant fusion peptabody, or a part thereof, can be synthesized by standard chemical techniques, for example, the phosphotriester method or via automated synthesis methods and PCR methods. The purified and isolated DNA sequence encoding the isolated and recombinant fusion peptabody according to the invention may also be produced by enzymatic techniques. Thus, restriction enzymes, which cleave nucleic acid molecules at predefined recognition sequences can be used to isolate nucleic acid sequences from larger nucleic acid molecules containing the nucleic acid sequence, such as DNA (or RNA) that codes for the isolated and recombinant fusion peptabody or a part thereof.

Also disclosed are variants of the aforementioned sequences, that is nucleotide sequences that vary from the reference sequence by conservative nucleotide substitutions, whereby one or more nucleotides are substituted by another with same characteristics.

The vector can be capable of replication in eukaryotes, prokaryotes or in eukaryotes and prokaryotes. It can be a vector capable of integration into the genome of the host cell (e.g. bacteriophage lambda) or a vector which is present extrachromosomally (e.g. a plasmid). Preferably, the vector is a plasmid.

Further, the vector can be an expression vector, i.e. a vector in which the isolated and purified DNA sequence is operably to a promoter. This means that the linked isolated and purified DNA sequence encoding the isolated and recombinant fusion peptabody of the present invention is under control of a suitable regulatory sequence which allows expression, i.e. transcription and translation of the inserted isolated and purified DNA sequence. Suitable expression vectors for prokaryotic host cells are described in Sambrook et al., Molecular Cloning; a Laboratory Manual, 2nd Edition (1989), Cold Spring Harbor Laboratory Press, especially in Chapters 1-4 and 17. Suitable vectors for expression of cloned genes in mammalian cells are described in Sambrook et al., Chapter 16. Suitable vectors can be chosen or constructed, containing appropriate regulatory sequences, including promoter sequences, terminator sequences, polyadenylation sequences, marker genes and other sequences as appropriate.

A wide variety of host/expression vector combinations may be employed in expressing the DNA sequences of this invention. Useful expression vectors, for example, may consist of segments of chromosomal, non-chromosomal and synthetic DNA sequences. Suitable vectors include derivatives of SV40 and known bacterial plasmids, e. g., E. coli plasmids col EI, pCRI, pBR322, pMB9 and their derivatives, plasmids such as RP4; phage DNAs, e. g., the numerous derivatives of phage X, e. g., NM989, and other phage DNA, e. g., M13 and filamentous single stranded phage DNA; yeast plasmids such as the 2u plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Any of a wide variety of expression control sequences (i.e. sequences that control the expression of a DNA sequence operatively linked to it) may be used in these vectors to express the DNA sequences of this invention. Such useful expression control sequences include, for example, the early or late promoters of SV40, CMV, vaccinia, polyoma or adenovirus, the lac system, the trp system, the TAC system, the TRC system, the LTR system, the major operator and promoter regions of phage X, the control regions of fd coat protein, the promoter for 3-phosphoglycerate kinase or other glycolytic enzymes, the promoters of acid phosphatase (e. g., Pho5), the promoters of the yeast-mating factors, and other sequences known to control the expression of genes of prokaryotic or eukaryotic cells or their viruses, and various combinations thereof.

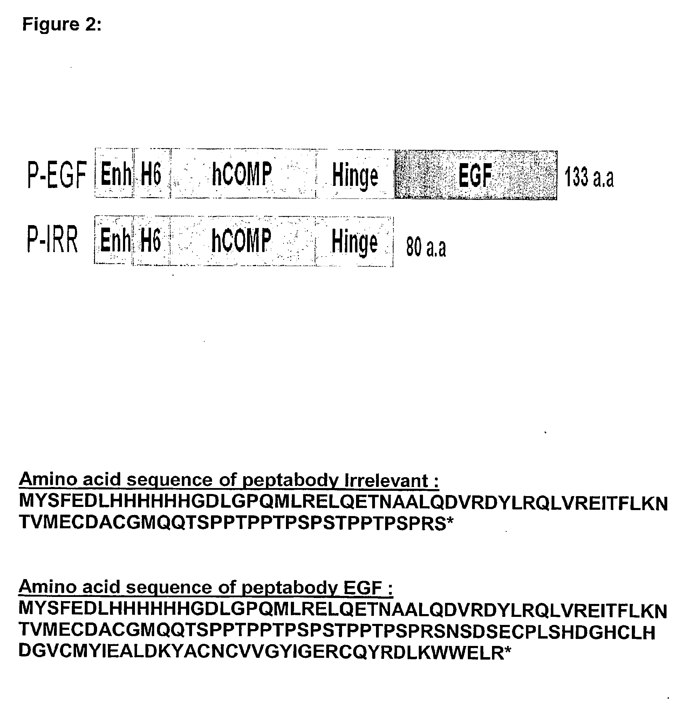

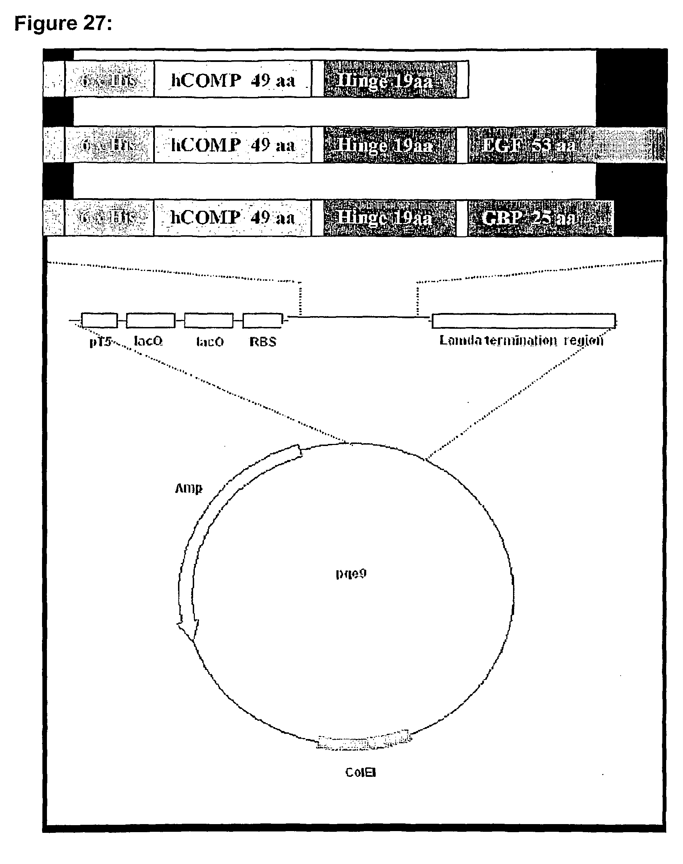

Specific examples of expression vectors of the present invention are the E. coli vectors pMS238-5-TGF, pMS238-5-225, pMS238-225-5, pMS240-5-225, pMS242-5-5 KDEL, pMS238-5-5 and pMS246-5-5 KDEL. Preferably, the expression vector is pQE-09 (Fig 27). The nucleotide sequences of plasmid fragments encoding polypeptides of the present invention and the corresponding amino acid sequences are shown in Figure 2, Figures 23, 24 and in Figure 27 as well as in the enclosed SEQ ID N° 1 and 2 for peptabody EGF (p-EGF) and SEQ ID N° 3 and 4 for p-GBP.

A further subject matter of the present invention is a host cell capable of expressing the isolated and purified DNA molecule as defined above, particularly a host cell which is stably transformed with an expression vector containing said DNA molecule.

A wide variety of unicellular host cells are useful in expressing the DNA sequences of this invention. These hosts may include well known eukaryotic and prokaryotic hosts, such as strains of E. coli, Pseudomonas, Bacillus, Streptomyces, fungi such as yeasts, and animal cells, such as CHO, YB/20, NSO, SP2/0, RI. I, B-W and L-M cells, African Green Monkey kidney cells (e. g., COS 1, COS 7, BSC1, BSC40, and BMT10), insect cells (e. g., Sf9), and human cells and plant cells in tissue culture. Preferably, the host cell is a bacterial cell, more preferably an E. coli cell.

It will be understood that not all vectors, expression control sequences and hosts will function equally well to express the DNA sequences of this invention. Neither will all hosts function equally well with the same expression system. However, one skilled in the art will be able to select the proper vectors, expression control sequences, and hosts without undue experimentation to accomplish the desired expression without departing from the scope of this invention. For example, in selecting a vector, the host must be considered because the vector must function in it. The vector's copy number, the ability to control that copy number, and the expression of any other proteins encoded by the vector, such as antibiotic markers, will also be considered.

In selecting an expression control sequence, a variety of factors will normally be considered. These include, for example, the relative strength of the system, its controllability, and its compatibility with the particular DNA sequence to be expressed, particularly as regards potential secondary structures. Suitable unicellular hosts will be selected by consideration of, e.g., their compatibility with the chosen vector, their secretion characteristics, their ability to fold proteins correctly, and their fermentation requirements, as well as the toxicity to the host of the product encoded by the DNA sequences to be expressed, and the ease of purification of the expression products.

Considering these and other factors a person skilled in the art will be able to construct a variety of vector/expression control sequence/host combinations that will express the DNA sequences of this invention on fermentation or in large-scale animal culture.

Host cells are transformed with the above-described expression or cloning vectors for Peptabody production and cultured in conventional nutrient media modified as appropriate for inducing promoters, selecting transformants, or amplifying the Peptabody production.

The host cells used to produce the isolated and recombinant fusion peptabody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430 or WO 87/00195 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN(TM) drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

A further subject matter of the present invention is a pharmaceutical composition comprising as an active substance a pharmaceutically effective amount of the isolated and recombinant fusion peptabody as defined above, optionally in combination with pharmaceutically acceptable carriers, diluents and adjuvants. Preferably, the pharmaceutical composition of the present invention comprises pharmaceutically acceptable carriers, diluents and adjuvants. Such acceptable carriers, diluents and adjuvants should be non-toxic and should not interfere with the efficacy of the active ingredient. This pharmaceutical composition is preferably used as an agent for the preparation of a medicament for the treatment or prevention of cancer, for the inhibition of cell proliferation, more preferably as an anti-tumor agent, i.e. an agent for inhibiting the growth of a tumor.

The pharmaceutical composition can be in any suitable form, e.g. in the form of a solution, suspension, powder, lyophilisate, ointment or tincture. The composition can be administered by any suitable method, e.g. per injection (systemically or locally) or topically. The precise nature of the carrier or other material will depend on the route of administration, which may be oral, topical, or by injection, e.g. intravenous or intradermal.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil.

Physiological saline solution, dextrose or other saccharide solution or glycols such as ethylene glycol, propylene glycol or polyethylene glycol may be included.

For intravenous, injection, or injection at the site of affliction, the active ingredient will be in the form of a parenterally acceptable aqueous solution which is pyrogenfree and has suitable pH, isotonicity and stability. Those of relevant skill in the art are well able to prepare suitable solutions using, for example, isotonic vehicles such as Sodium Chloride Injection, Ringer's Injection, Lactated Ringer's Injection.

Preservatives, stabilisers, buffers, antioxidants and/or other additives may be included, as required.

The respective pharmaceutically effect amount can depend on the specific patient to be treated, on the disease to be treated and on the method of administration. Further, the pharmaceutically effective amount depends on the specific polypeptide used, especially if the polypeptide additionally contains a cytotoxic component or not. The treatment usually comprises a multiple administration of the pharmaceutical composition, usually in intervals of several hours, days or weeks. The pharmaceutically effective amount of a dosage unit of the polypeptide usually is in the range of 0.001 ng to 100 µg per kg of body weight of the patient to be treated.

Pharmaceutical compositions used in accordance with the present invention are prepared for storage by mixing a pharmaceutically effective amount of the isolated and recombinant fusion peptabody as defined above having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN(TM), PLURONICS(TM) or polyethylene glycol (PEG).

The pharmaceutical composition or formulation herein may also contain more than one active compound as necessary for the particular indication being treated, preferably those with complementary activities that do not adversely affect each other. For example, it may be desirable to further provide antibodies which bind to EGFR, ErbB2 (e.g. an antibody which binds a different epitope on ErbB2), ErbB3, ErbB4, or vascular endothelial factor (VEGF) in the one formulation. Alternatively, or additionally, it will become apparent that the pharmaceutical composition may be administered alone or in combination with other treatments, therapeutics or agents, either simultaneously or sequentially dependent upon the condition to be treated. Preferably, the pharmaceutical composition may further comprise a chemotherapeutic agent, cytotoxic agent, cytokine, growth inhibitory agent, anti-hormonal agent, EGFR-targeted drug, anti-angiogenic agent, anti-cancer agents, immune modulators and/or cardioprotectant. Such molecules are suitably present in combination in amounts that are effective for the purpose intended.

More generally these anti-cancer agents may be tyrosine kinase inhibitors or phosphorylation cascade inhibitors, post-translational modulators, cell growth or division inhibitors (e. g. anti-mitotics), or signal transduction inhibitors. Other treatments or therapeutics may include the administration of suitable doses of pain relief drugs such as non-steroidal anti-inflammatory drugs (e. g. aspirin, paracetamol, ibuprofen or ketoprofen) or opiates such as morphine, or anti-emetics. The composition can be administered in combination (either sequentially (i. e. before or after) or simultaneously) with tyrosine kinase inhibitors (including, but not limited to AG1478 and ZD1839, STI571, OSI-774, SU-6668), doxorubicin, temozolomide, cisplatin, carboplatin, nitrosoureas, procarbazine, vincristine, hydroxyurea, 5-fluoruracil, cytosine arabinoside, cyclophosphamide, epipodophyllotoxin, carmustine, lomustine, and/or other chemotherapeutic agents. Thus, these agents may be anti EGFR specific agents, or tyrosine kinase inhibitors such as AG1478, ZD1839, ST1571, OSI-774, or SU-6668 or may be more general anti-cancer and anti-neoplastic agents such as doxorubicin, cisplatin, temozolomide, nitrosoureas, procarbazine, vincristine, hydroxyurea, 5-fluoruracil, cytosine arabinoside, cyclophosphamide, epipodophyllotoxin, carmustine, or lomustine. In addition, the pharmaceutical composition may be administered with hormones such as dexamethasone, immune modulators, such as interleukins, tumor necrosis factor (TNF) or other growth factors or cytokines which stimulate the immune response and reduction or elimination of cancer cells or tumors.

The active ingredients or agents may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi permeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and [gamma] ethyl-L-glutamate, non-degradable ethylene-vinyl acetate, degradable lactic acid-glycolic acid copolymers such as the LUPRON DEPOT(TM) (injectable microspheres composed of lactic acid-glycolic acid copolymer and leuprolide acetate), and poly-D-(-)-3-hydroxybutyric acid.

The formulations to be used for in vivo administration must be sterile. This is readily accomplished for example by filtration through sterile filtration membranes.

It is contemplated that, the pharmaceutical composition comprising as an active substance a pharmaceutically effective amount of the isolated and recombinant fusion peptabody as defined above may be used to treat various diseases or disorders. Generally, the disease or disorder to be treated is cancer. Also disclosed is a method of treating or preventing cancer that expresses epidermal growth factor receptors (ErbB-expressing cancer), preferably for cancer characterized by excessive activation of an ErbB receptor or for cancer which "overexpresses" an ErbB receptor.

Examples of cancer to be treated herein include, but are not limited to, carcinoma, lymphoma, blastoma, sarcoma, and leukemia or lymphoid malignancies. More particular examples of such cancers include carcinoma, lymphoma, blastoma, sarcoma, liposarcoma, neuroendocrine tumor, mesothelioma, schwanoma, meningioma, adenocarcinoma, melanoma, leukemia, lymphoid malignancy, squamous cell cancer, epithelial squamous cell cancer, lung cancer, small-cell lung cancer, non-small cell lung cancer, adenocarcinoma of the lung, squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, a tumor of the biliary tract, and head and neck cancer.

More preferably, the cancer to be treated herein is head cancer, neck cancer, bladder cancer or melanoma.

Also disclosed is a method for inducing apoptosis and/or necrosis, comprising contacting a cell with the isolated and recombinant fusion peptabody of the present invention. The cell to be contacted is preferably a cancer cell as described above.

Also disclosed is a method for inhibiting cell proliferation, comprising contacting a cell with the isolated and recombinant fusion peptabody of present invention. The cell to be contacted is preferably a cancer cell as described above.

For the prevention or treatment of disease and especially cancer, the appropriate dosage of the isolated and recombinant fusion peptabody will depend on the type of disease to be treated, as defined above, the severity and course of the disease, whether the isolated and recombinant fusion peptabody is administered for preventive or therapeutic purposes, previous therapy, the patient's clinical history and response to the isolated and recombinant fusion peptabody, and the discretion of the attending physician. The isolated and recombinant fusion peptabody is suitably administered to the patient at one time or over a series of treatments. Depending on the type and severity of the disease, about 1 µg/kg to 15 mg/kg (e.g. 0.1-20 mg/kg) of peptabody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The preferred dosage of the isolated and recombinant fusion peptabody will be in the range from about 0.005 mg/kg to about 1.0 mg/kg. Thus, one or more doses of about 0.05 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the isolated and recombinant fusion peptabody). An initial higher loading dose, followed by one or more lower doses may be administered. An exemplary dosing regimen comprises administering an initial loading dose of about 0.4 mg/kg, followed by a weekly maintenance dose of about 0.2 mg/kg of the isolated and recombinant fusion peptabody. However, other dosage regimens may be useful. The progress of this therapy is easily monitored by conventional techniques and assays.

Due to the high specificity of the isolated and recombinant fusion Peptabodies of the invention, they can also be used in gene therapy, e.g., to target certain receptors for activation or deactivation; or, if connected to toxic compounds, for their destruction. Cell therapy based on the use of a host cell previously transfected with an expression vector described herein may also be envisaged.

Also disclosed is a method of diagnosing cancer, comprising administering to a subject the isolated and recombinant fusion peptabody as defined above, optionally in combination with pharmaceutically acceptable carriers, diluents and adjuvants.

Another object of the present invention is to provide a kit for diagnosing cancer that expresses epidermal growth factor receptors in a human patient, said kit comprising the isolated and recombinant fusion peptabody bearing at least one detection moiety as defined above, optionally with reagents and/or instructions for use.

Diagnostic assays and kits for in vitro assessment and evaluation of EGFR status, particularly with regard to aberrant expression of EGFR, may be utilized to diagnose, evaluate and monitor patient samples including those known to have or suspected of having cancer, a precancerous condition, a condition related to hyperproliferative cell growth or from a tumor sample. The assessment and evaluation of EGFR status is also useful in determining the suitability of a patient for a clinical trial of a drug or for the administration of a particular chemotherapeutic agent or particularly, the isolated and recombinant fusion peptabody of the present invention, including combinations thereof, versus a different agent.

The labels or detection moieties most commonly employed for these studies are radioactive elements, enzymes, chemicals which fluoresce when exposed to ultraviolet light, and others.

It is another embodiment of the invention to provide a kit for treating cancer that expresses epidermal growth factor receptors in a human patient, said kit comprising the isolated and recombinant fusion peptabody of the present invention, optionally with reagents and/or instructions for use.

The kit of the present invention may further comprise a separate pharmaceutical dosage form comprising an additional anti-cancer agent selected from the group consisting of chemotherapeutic agents, anti-epidermal growth factor receptors antibodies, radioimmunotherapeutic agents, and combinations thereof.

Generally, the Kit comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is the isolated and recombinant fusion peptabody of the present invention. The label or package insert indicates that the composition is used for treating the condition of choice, such as cancer. In one embodiment, the label or package inserts indicates that the composition comprising the isolated and recombinant fusion peptabody of the present invention which binds EGFR can be used to treat cancer which expresses an ErbB receptor selected from the group consisting of epidermal growth factor receptor (EGFR), ErbB3 and ErbB4, preferably EGFR. In addition, the label or package insert may indicate that the patient to be treated is one having cancer characterized by excessive activation of an ErbB receptor selected from EGFR, ErbB3 or ErbB4.

Alternatively, or additionally, the Kit may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Peptabodies of the prior art have been synthesized by several ways.

Due to the relatively short length of the domain capable of binding to an acceptor (ligand), Peptabodies of the prior art have preferably been chemically synthesized, e.g. via solid phase synthesis.

Alternatively, a mixed process has been reported. A part of the inventive unit is expressed by a suitable host and is connected with a chemically synthesized part. For example, the oligomerizing part is synthesized by a microorganism and is elongated by chemical synthesis to add the domain capable of binding to an acceptor.

Genetic engineering was not yet reported for ligands having complex structures and sizes like polypeptides or proteins. Only small ligands no longer than hexapeptides have been obtained by expressing the inventive unit as a fusion protein. Most of them are produced as insoluble form suggesting the difficulty to produce complex structures on the inventive units.

The isolated and recombinant fusion peptabody of the present invention is preferably produced by Genetic engineering methods. An expression vector as disclosed above is constructed comprising an expression cassette for the complete DNA sequence encoding the isolated and recombinant fusion peptabody that is expressed in a suitable host cell as described above. Suitable expression cassettes can be prepared by standard techniques in genetic engineering.

Beside the information necessary for the synthesis of the desired isolated and recombinant fusion peptabody, the expression cassette may additionally contain a signal sequence that forces the secretion of the protein produced.

The present invention provides a method for producing the isolated and recombinant fusion peptabody of the present invention, comprising the steps of:
a) constructing an isolated and purified DNA molecule encoding the isolated and recombinant fusion peptabody as described above,
b) allowing expression of said isolated and purified DNA molecule in a cell system under suitable conditions,
c) recovering the isolated and recombinant fusion peptabody.

Peptabody is a multimeric molecule which is formed by multimerization of cartilage oligomeric matrix proteins. This event occurs spontaneously when monomers are mixed together under appropriate conditions (low denaturing conditions). However, the efficiency of pentamer formation can vary according to physico-chemical conditions and the concentration of monomers. In addition, the complexity of the ligands, particularly ligands containing disulphide bridges, can cause the formation of aggregates or multimers of peptabodies.

As described above, the cell expression system or host cell is a eukaryotic or a prokaryotic cell. Preferably, the cell expression system is a prokaryotic cell.

Suitable conditions of step b) consist in culturing the cell expression system at a temperature between 10-40 °C during 2-40 hours and preferably at a temperature of 37°C during 8-16 hours.

Further, step c) is achieved by extraction of the isolated and recombinant fusion peptabody from the cell expression system subsequently followed by purification and refolding steps.

Recombinant fusion peptabody can be extracted and recovered either from the culturing medium, when the recombinant fusion peptabody is secreted, or extracted from the cell expression system when the recombinant fusion peptabody is not secreted, and purified by art-known techniques such as high performance liquid chromatography, gel electrophoresis, affinity chromatography, ultrafiltration, ion exchange and the like. The actual conditions used to purify a particular recombinant fusion peptabody will depend, in part, on factors such as net charge, hydrophobicity, hydrophilicity, etc. and will be apparent to those skilled in the art.

For affinity chromatography purification, any antibody which specifically binds to the recombinant fusion peptabody or to the His tag may be used. Other affinity molecules such as Ni²⁺-nitrilotriacetic linked to agarose beads and which bind specifically to the His tag are also envisioned in the present invention.

Purification is carried out in the presence of reducing agents and results in the elimination of contamination.

The refolding step is carried out by direct dilution in refolding buffer and further comprises serial dialysis. A crucial point is to perform a rapid dilution of denatured Peptabodies in renaturing solution.

Recovering of the isolated and recombinant fusion peptabody was carried out at a low concentration in order to avoid aggregate formation. Preferably, the direct dilution in refolding buffer leads to a final concentration of the isolated and recombinant fusion peptabody below 300 nM and most preferably below 100 nM as shown in Example 4.

The serial dialysis comprises at least 2 different dialysis but preferably at least 3 or 4 and even more preferably at least 5 different dialysis buffers as shown in Example 4. However, one skilled in the art will be able to select other dialysis sequences or serials without departing from the scope of this invention.

The refolding step consists in the oxidation of the isolated and recombinant fusion peptabody before its concentration.

The Peptabody, which is produced according to the present invention, is particularly stable. Stability of the peptabody molecule means the maintenance of a stable soluble form of the molecule without switching to insoluble forms or formation of aggregates or formation of multimers of Peptabodies or loss of biological activity.

"Protein production increasing activity" refers to an activity of the purified and isolated enhancer sequence defined as follows: after having been introduced under suitable conditions into a eukaryotic or prokaryotic host cell, the sequence is capable of increasing protein production levels in cell culture as compared to a culture of cell transfected without said enhancer sequence.

Those skilled in the art will appreciate that the invention described herein is susceptible to variations and modifications other than those specifically described. The present disclosure is therefore to be considered as in all aspects illustrated and not restrictive, the scope of the invention being indicated by the appended Claims.

The foregoing description will be more fully understood with reference to the following Examples. Such Examples, are, however, exemplary of methods of practising the present invention and are not intended to limit the scope of the invention.

### EXAMPLES

### Example 1:

### Construction and production of Peptabodies anti-EGFR:

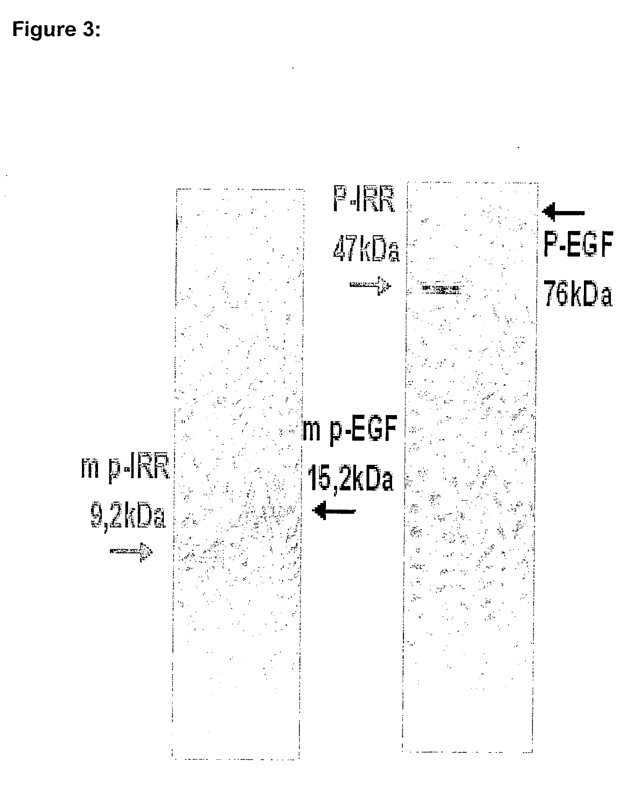

The gene encoding the human epidermal growth factor was amplified by PCR from a plasmid containing the full-length gene, digested with appropriate enzymes and inserted into a plasmid containing the peptabody gene. Peptabodies are produced in bacteria into the cytoplasm as inclusion bodies by induction at 37°C overnight. E. coli cells are lysed in denatured solution (in 8M urea) at room temperature. After centrifugation, peptabody molecules are purified by affinity chromatography and refolded by direct dilution in refolding buffer (see Fig. 3).

### Binding of Peptabodies EGF on EGF receptor of tumor cells:

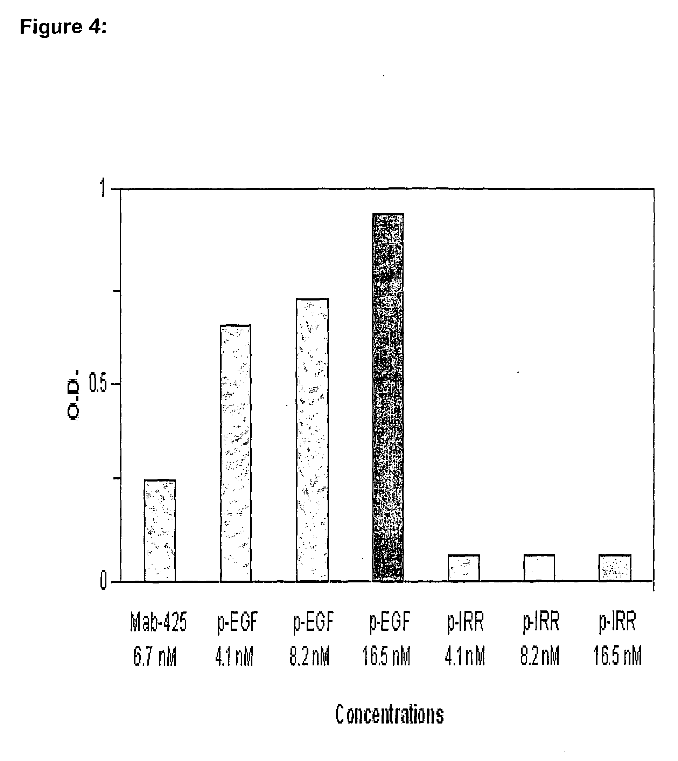

Binding assay was performed on A431 cells using monoclonal antibody Mab425 (used in therapy as Retuximab ®), peptabody EGF and peptabody Irrelevant. Briefly, 10 000 cells were seeded in 96 well plate for 10 hours in culture media/10% FCS. After removal of media, Peptabodies or monoclonal antibody were incubated at different concentrations with adherent cells for 90 minutes on ice. After washing, revelation was done using peroxidase conjugated polyclonal anti-Fab or peroxidase-conjugated monoclonal anti-His for respectively MAb 425 and Peptabodies (see Fig. 4).

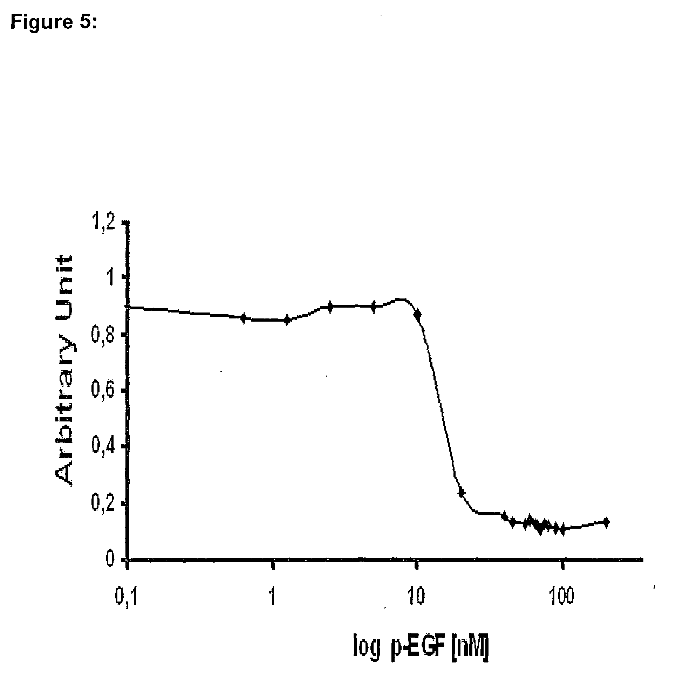

A competition assay was performed between Mab425 at 6.67 nM and increasing amounts of Peptabody EGF (0.1 to 200 nM) demonstrating that Mab425 and Peptabody EGF compete for the same binding site, EGF receptor (see Fig. 5).

### Example 2:

### In vitro actions on different cancer cell lines

The evaluation of Peptabody effect on human cancer cells was performed using a viability assay. Briefly, 2500 cells were seeded on 96 well plates in DMEM with 10% of foetal calf serum. After one day, medium was removed and replaced by optimem in presence of Peptabodies. At different times, a plate was revealed using MTT assay to evaluate the number of cells.

### Epidermoïd cancer cells A431:

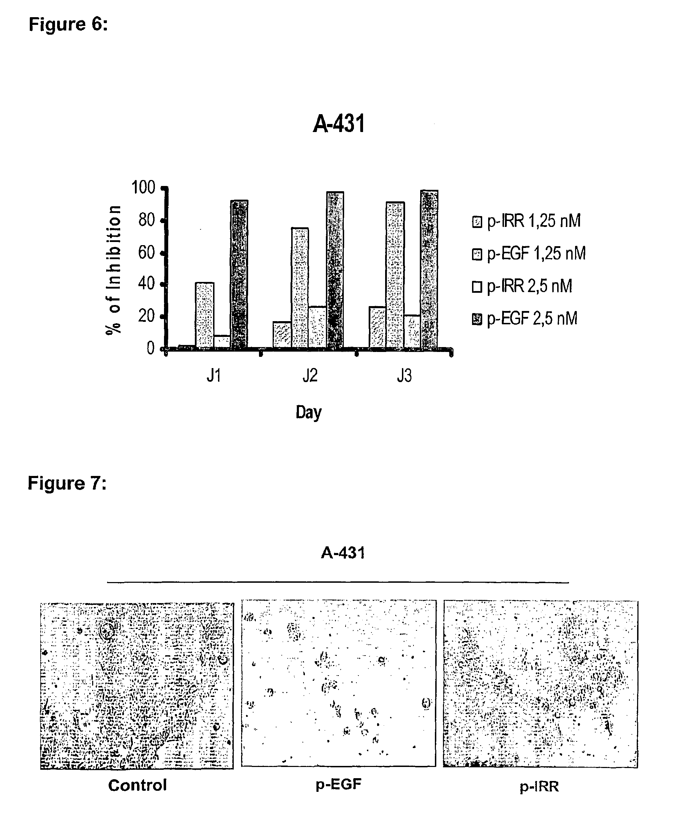

Fig. 6: shows a viability assay of human epidermoïd cancer cells A431 in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 7: is a visualization of *in vitro* culture of epidermoïd cancer cells A431 in DMEM without serum after 2 days of treatment with peptabody EGF or peptabody Irrelevant p-IRR.

### Human prostate cancer cells DU145:

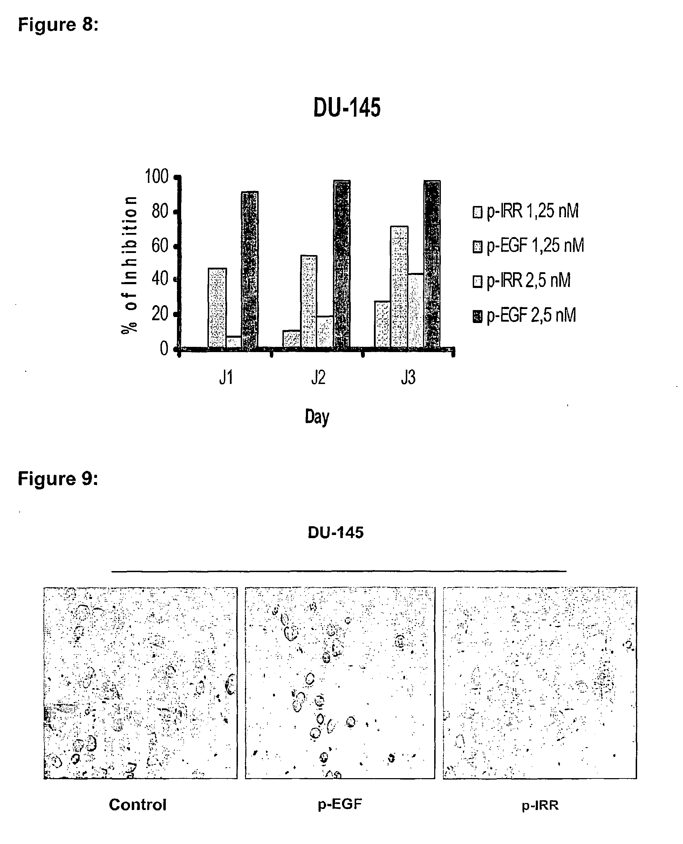

Fig. 8: shows a viability assay of human prostate cancer cells DU145 in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 9: is a visualization of *in vitro* culture of prostate cancer cells DU-145 in DMEM without serum after 2 days of treatment with peptabody EGF or peptabody Irrelevant p-IRR.

### Human prostate cancer cells LNCaP:

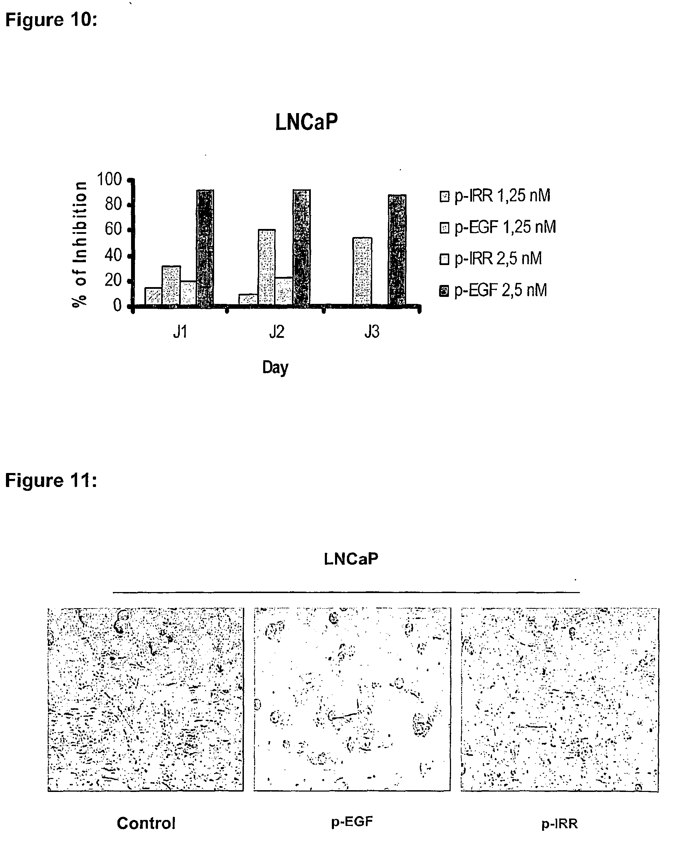

Fig. 10: shows a viability assay of human prostate cancer cells LNCaP in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 11: is a visualization of *in vitro* culture of prostate cancer cells LNCaP in DMEM without serum after 2 days of treatment with peptabody EGF or peptabody Irrelevant p-IRR.

### Human prostate cancer cells PC-3:

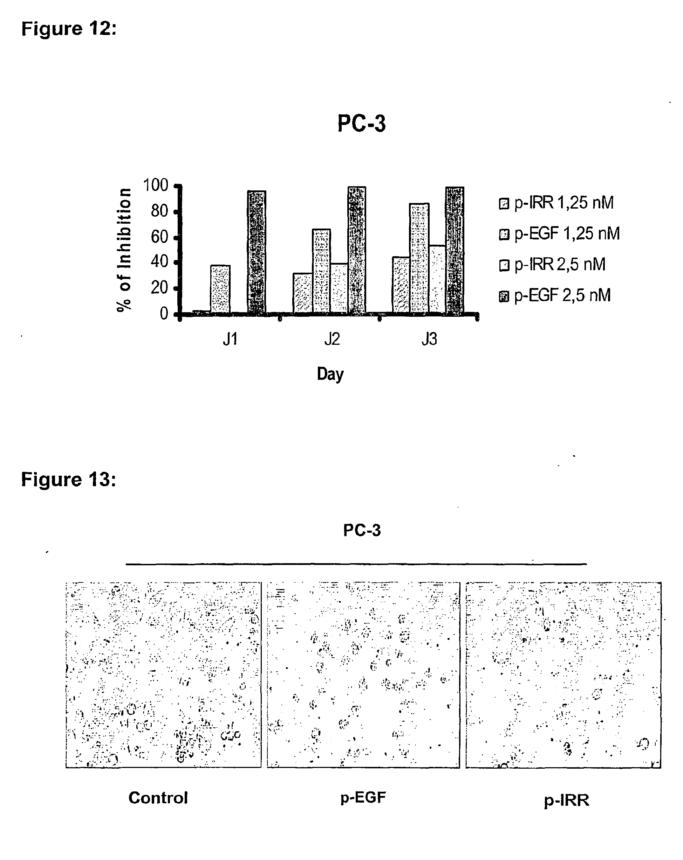

Fig. 12: shows a viability assay of human prostate cancer cells PC-3 in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 13: is a visualization of *in vitro* culture of prostate cancer cells PC-3 in DMEM without serum after 2 days of treatment with peptabody EGF or peptabody Irrelevant p-IRR.

### Human breast cancer cell MCF-7:

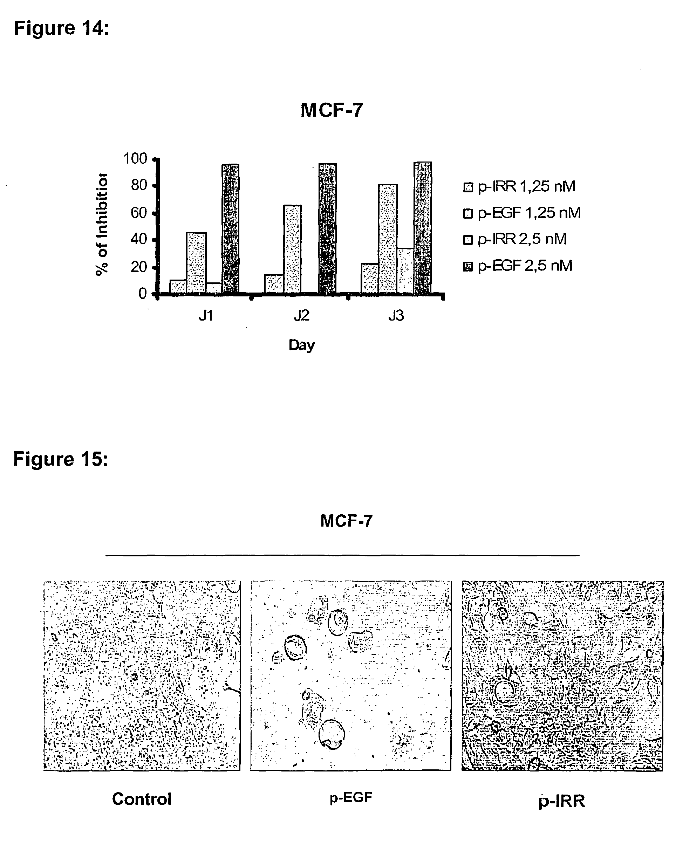

Fig. 14: shows a viability assay of human breast cancer cells MCF-7 in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 15: is a visualization of in vitro culture of breast cancer cells MCF-7 in DMEM with foetal calf serum after 2 days of treatment with peptabody EGF or peptabody Irrelevant p-IRR.

### Control on human normal muscle cells:

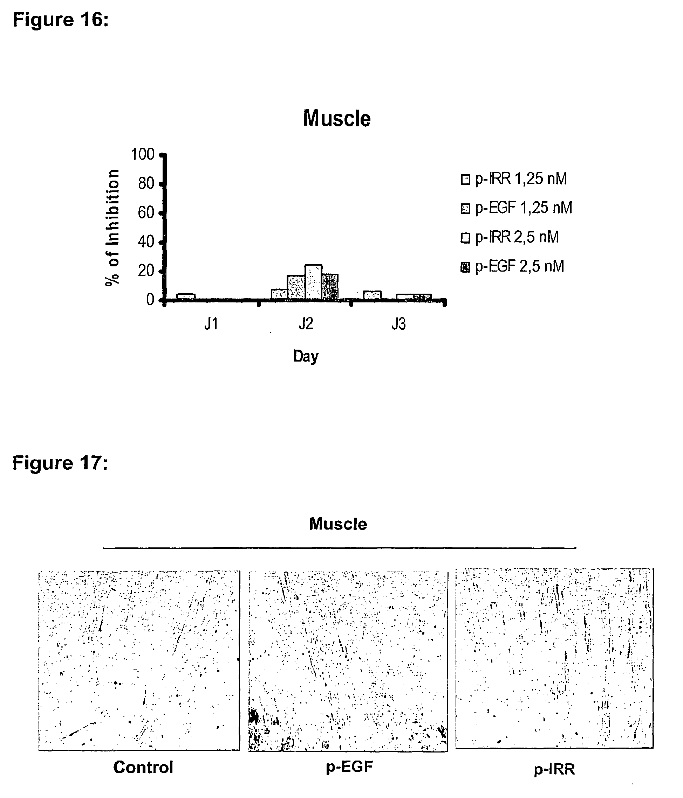

Fig. 16: shows a viability assay of human normal muscle cells in presence of different concentrations of Peptabodies EGF (p-EGF) or Irrelevant (p-IRR) at one, two and three days after treatment. The percentage (%) of inhibition is calculated by the ratio of viable cells in comparison to the control. Fig. 17: is a visualization of *in vitro* culture of muscle cells in DMEM without serum after 2 days of treatment with peptabody EGF or peptabody Irrelevant p-IRR.

### In vitro actions on different cancer cell lines:

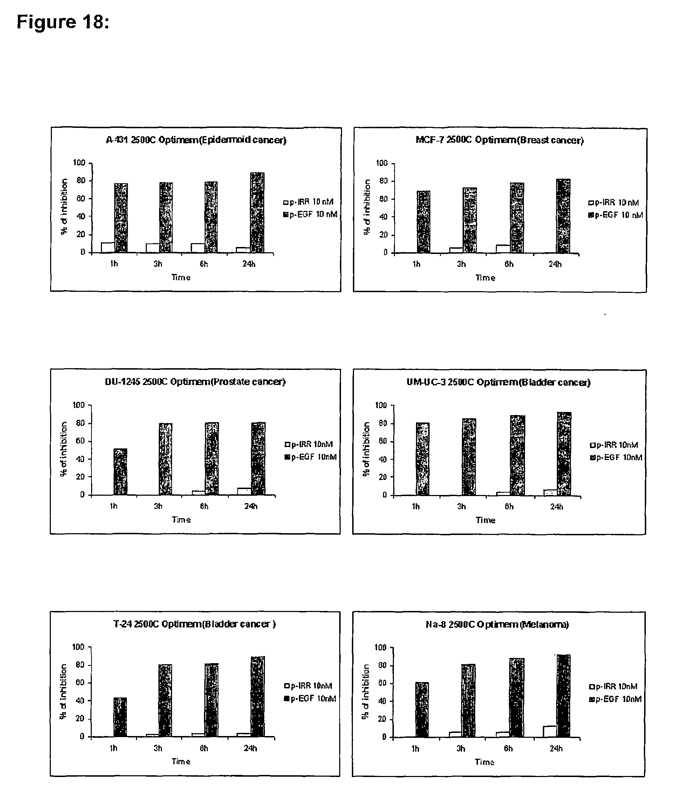

Evaluations of Peptabody effect on human cancer cells was performed using a viability assay. Briefly, 2500 cells were seeded on 96 well plates in DMEM with 10% of foetal calf serum. After one day, medium was removed and replaced by optimem in presence of Peptabodies. Effect of Peptabodies at 10 nM was assessed on human cancer cells at different times within one day (see Fig. 18). In addition, lower concentrations of peptabodies were evaluated for 3 days. Plates were revealed at each time using MTT assay to evaluate the number of cells.

### In conclusion, a strong inhibition of the cell growth is observed (see Fig. 7, 9, 11, 13 and 15) in the presence of the peptabody p-EGF compared to the irrelevant peptabody, p-IRR (blank).

### Example 3:

Detection of apoptosis. A-431 cancer cells (5x10⁵ in 500µl per well) were incubated in DMEM medium. After 24h the medium was replaced with 500µl of the serum free medium OPTIMEM containing 10nM of p-EGF or p-IRR. At indicated time points between 0 and 360 min. (T₀, T₃₀, T₆₀, T₁₈₀, T₃₆₀) adherent and floating cells were collected, pooled and analyzed for apoptosis using an annexin V-FITC apoptosis detection kit (Apotech, Switzerland) that detects phosphatidylserine on the outer surface of the cell membrane. Briefly, cells were washed once each with PBS and staining buffer and stained at room temperature for 15 minutes with 50 µl of annexin V-FITC [5µg/ml]. Stained cell pellets were washed once each with staining buffer and with FACS buffer (1xPBS, 5% FCS, 0.02% NaN₃), resuspended in 200µl of FACS buffer, counted and analysed by FACS.

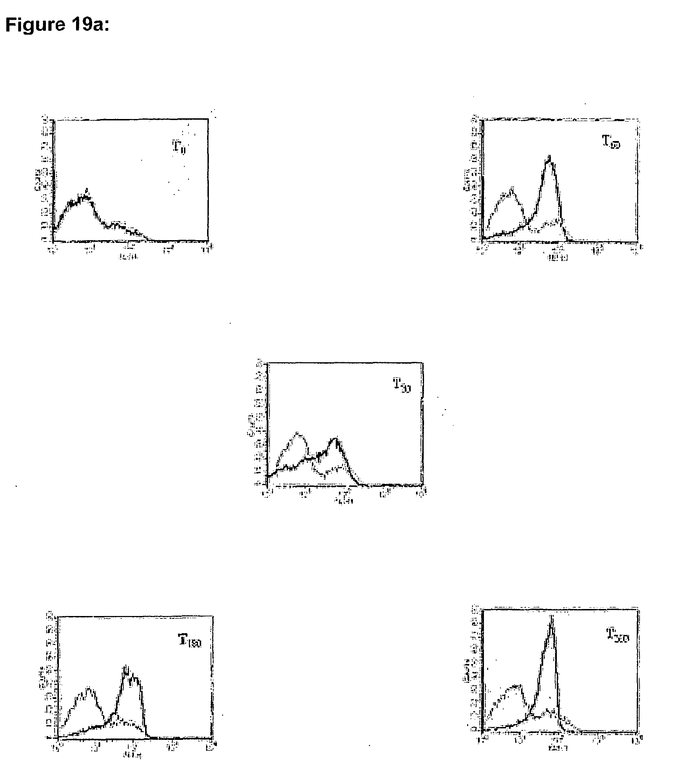

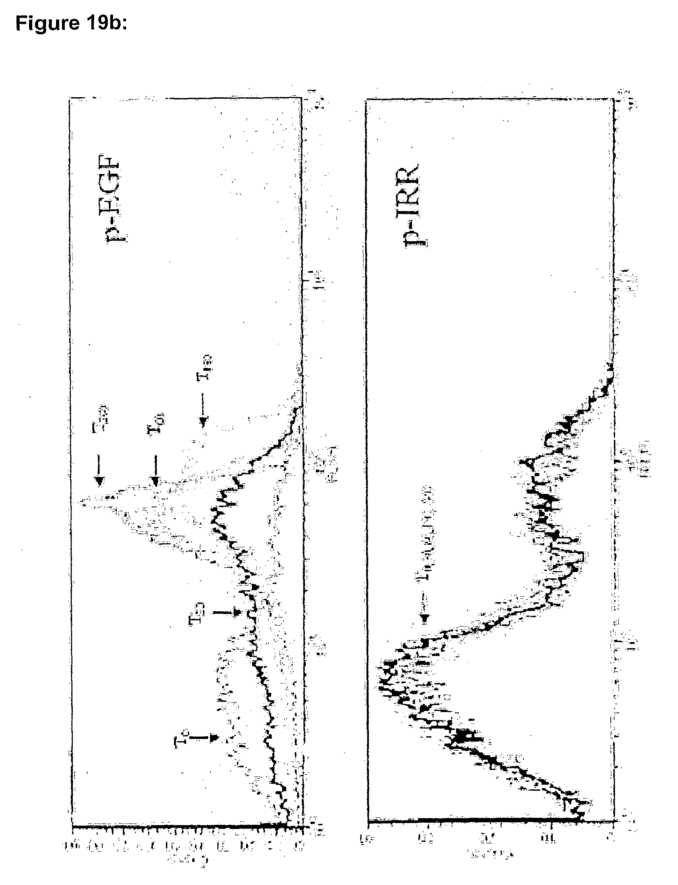

Peptabody EGF clearly induced apoptosis of A431 cancer cells after 30 minutes whereas peptabody IRR had no effect (see Fig. 19a and Fig 19b).

### Example 4:

### Peptabody production and refolding protocol

- Production in bacteria: E. coli TG1 strain containing plasmid encoding for Peptabodies was used for production of recombinant proteins. From an overnight pre-culture, a fresh culture was started by 1/100 dilution in 250ml of 2xTY medium supplemented with 100ug/mL ampicillin at 37°. At 0.6 O.D. (600 nm), culture was induced with 250mM isopropyl β-D-thiogalactoside (IPTG).
- Purification: After 16h of induction, bacteria were harvested by centrifugation and pellet was resuspended in 25mL of denaturing solution (8M urea, 1xPBS, 10mM β-mercaptoethanol at pH 7,4) during 2h at room temperature. The insoluble material was pelleted by centrifugation at 10000g for 10min and supernatant was mixed with 250ul of Ni²⁺-NTA agarose beads (Qiagen). After 2h at room temperature under rotation, the resin was washed three times with a washing solution (5M urea, 1xPBS, 10mM β-mercaptoethanol, 20mM Imidazole at pH 7.4). Proteins were eluted in 2.5mL with elution buffer (5M urea, 1xPBS, 10mM β-mercaptoethanol, 150mM Imidazole at pH 7.4).
- Refolding:
   ● The 1.25mg-eluted proteins are quickly diluted in 250 ml of refolding buffer (50mM Tris, 50mM NaCl, 10mM β-mercaptoethanol, 0.1% Triton-X100 at pH=8.2) to a final concentration inferior to 5µg/ml, and then maintained for at least 6 hours in these conditions.
   • Proteins were then dialyzed using the following order of buffers (bath of 4L):
   • Dialysis buffer 1: 50mM Tris, 50mM NaCl, 1 mM P-mercaptoethanol 0.1 % Triton-X100 at pH=8.2 during 6h.
   • Dialysis buffer 2: 50mM Tris, 50mM NaCl, 0.1 % Triton-X100, pH=8.2, overnight.
   • Dialysis buffer 3: 50mM Tris, 50mM NaCl, 0.01 % Triton-X100, pH=8.2, 4h.
   • Dialysis buffer 4: 50mM Tris, 50mM NaCl, 0.001 % Triton-X100, pH=8.2, 4h.
   • Dialysis buffer 5: 50mM Tris, 50mM NaCl, pH=8.2 overnight with bubbling bath.
   • The refolded proteins were concentrated using nitrogen stirred cell system and ultra centrifugal device (Millipore) to a final volume of 250 µl and conserved at - 80°C.

### Example 5:

### Materials

Primers were purchased from Microsynth (Switzerland).
Molecular biology reagents (Taq Polymerase, Ligase, Phosphatase) were purchased from Promega (Germany).
Expression vector pQE-09 and Ni-NTA agarose column were purchased from Qiagen (Germany).

### Production of Peptabodies anti-EGFR

### 1) Preparation of an expression vector

The expression vector pQE-09 was digested using Xhol and Notl restriction enzymes.

### 2) Peptabodies GBP/EGF/Irrelevant

A synthetic gene encoding for Growth-blocking peptide (GBP), an insect biogenic growth factor consisting of 25 amino acids from hemolymph of the *Pseudaletia separate,* was used to construct Peptabody GBP.

Gene encoding human epidermal growth factor was amplified by PCR from a plasmid containing the full-length gene.

GBP or EGF were inserted in a plasmid containing the gene encoding for decabody molecule digested with BgIII/Not1. The peptabody parts (Cartilage Oligomeric Matrix Protein -COMP-, Hinge, ligand) were amplified using PCR and specific primers. The resulting products were digested for their insertion in the pQE-09 expression vector.

### 3) Production of Peptabodies GBP/EGF/Irrelevant

Peptabodies were produced in bacteria into the cytoplasm as inclusion bodies and refolded as stable and soluble forms corresponding to pentamerized monomers.
- Gene encoding human epidermal growth factor was amplified by PCR from a plasmid containing the full-length gene.
- GBP or EGF genes were inserted into a plasmid containing peptabody gene modified according to the present invention.

The resulting products were digested for their insertion in the pQE-09 expression vector.

### 4) Production and purification of Peptabodies GBP/EGF/Irrelevant

Peptabodies are produced in bacteria into the cytoplasm as inclusion bodies by induction at 37°C overnight.

E. coli cells are lysed in 8M urea for two hours at room temperature. After centrifugation, lysate is mixed for 90 minutes with Ni-NTA agarose beads and Peptabody molecules are eluted with imidazole/Urea solution.

### 5) Refolding of Peptabodies GBP/EGF/Irrelevant

Peptabody molecules are refolded by direct dilution in refolding buffer (50 mM Tris HC1 pH 8.3, 0.01 Triton X100, 10mM β-mercaptoethanol) at a final concentration of 5 µg/ml (dilution must be superior to 100 fold) and dialyzed for 24 hours in 50 mM Tris HCl pH 8.3, 10mM β-mercaptoethanol and two days in 50 mM Tris HCl pH 8.3.

### 6) Final Purification of Peptabodies GBP/EGF/Irrelevant

Dialyzed Peptabodies are deposited on anionic exchange column and eluted using a salt gradient.

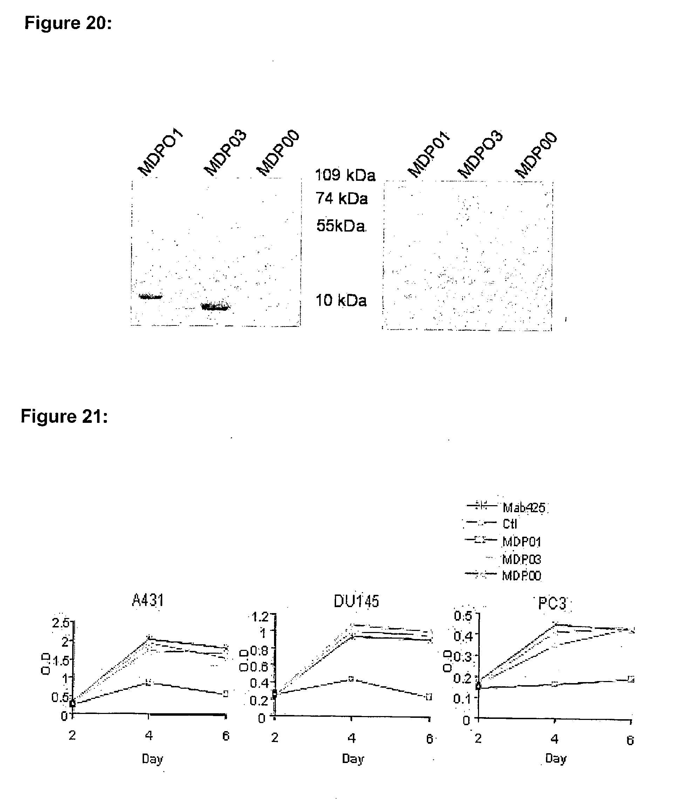

Results are shown in Fig. 20, representing a SDS-PAGE analysis of Peptabodies under non- or reducing conditions. MDP01 corresponds to a peptabody EGF, MDP03 is a peptabody GBP and MDP00 represents the irrelevant peptabody.

### Action of Peptabodies anti-EGFR on tumor cells

Refolded Peptabodies were tested on different tumor cells (A431: human epidermoïd cancer cells, DU145 and PC-3: human prostatic tumor cells) to evaluate their cytotoxic effect.

Results are shown in Fig. 21, representing a Cytotoxic assay on cancer cells using MTT method. Cancer cells were incubated with Peptabodies, or monoclonal antibodies specific to EGFR at a concentration of 2 µg/ml. Cancer cells were incubated with Peptabodies, or monoclonal antibodies specific to EGFR at a concentration of 2µg/ml. The percentages of binding of MDP01 and MDP03 on EGF receptor were significantly higher than MAb 425, a monoclonal antibody specific to EGF receptors (data not shown). Decrease of proliferation and increase of apoptosis on A-431 and DU145 cancer cell lines were observed in presence of Peptabodies whereas PC-3 were less sensitive for MDP03.

In conclusion, Peptabodies showed a stronger effect for mediating cancer cell death than monoclonal antibody 425.

### Example 6:

### Peptabody Enhancer sequences

Different versions of peptabody have been developed during the past. However, a large variability was observed in the peptabody/decabody production since most of them are not produced significantly.

Fig. 25: relates to the production of decabodies fused to different Enhances. A Coomassie blue staining of SDS PAGE of decabody production using a bacteria system is represented. A) corresponds to the insoluble fraction, B) corresponds to the soluble fraction.

Fig. 26: relates to the production of Peptabodies fused to different Enhancers. A Western blot analysis of peptabody production using a bacteria system is represented. The detection is performed with anti-His antibody from urea bacteria extract.

## Claims

1. An isolated and recombinant fusion peptabody, which binds to the epidermal growth factor receptor ErbB-1, ErbB-3 or ErbB-4, comprising:
(a) a portion of a humanized cartilage oligomer matrix polypeptide;
(b) a peptide enhancer sequence, having protein production increasing activity, located at the N terminus of the portion of the humanized cartilage oligomer matrix polypeptide and selected from the group consisting of: YSFE, YSFEDL and YSFEDLYRR;
(c) a portion of a hinge region of an immunoglobulin polypeptide located at the C terminus of the portion of the humanized cartilage oligomer matrix polypeptide;
(d) an epidermal growth factor receptor ligand located at the C terminus of the hinge region, comprising at least a motif having a three-dimensional structure,
and whereby said isolated and recombinant fusion peptabody is capable of inducing cellular death in a cell expressing epidermal growth factor receptor.

2. The isolated and recombinant fusion peptabody of claim 1, wherein the member of the epidermal growth factor receptor is ErbB-1.

3. The isolated and recombinant fusion peptabody of claims 1-2, wherein said isolated and recombinant fusion peptabody is multimeric.

4. The isolated and recombinant fusion peptabody of claims 1-3, wherein said epidermal growth factor receptor ligand is selected among the group of:
(a) an epidermal growth factor polypeptide or fragments thereof,
(b) a growth blocking peptide or fragments thereof,
(c) a TGF alpha polypeptide or fragments thereof,
(d) an amphiregulin polypeptide or fragments thereof,
(e) a heparin-binding epidermal growth factor-like polypeptide or fragments thereof,
(f) a betacellulin polypeptide or fragments thereof.

5. The isolated and recombinant fusion peptabody of claim 4, wherein said epidermal growth factor receptor ligand is present in its full-length sequence.

6. The isolated and recombinant fusion peptabody of claims 1-5, further comprising a polyhistidine tag sequence.

7. The isolated and recombinant fusion peptabody of claims 1-6, further comprising at least one effector region.

8. The isolated and recombinant fusion peptabody of claim 7, wherein the effector region comprises a cytotoxin.

9. The isolated and recombinant fusion peptabody of claim 7, wherein the effector region comprises a detection moiety.

10. The isolated and recombinant fusion peptabody of claim 9, wherein said detection moiety is fluorescent.

11. An isolated and purified DNA sequence encoding the isolated and recombinant fusion peptabody of any one of claims 1-7.

12. A vector comprising at least one copy of the isolated and purified DNA sequence of claim 11.

13. The vector of claim 12, further comprising a promoter operably linked to said isolated and purified DNA molecule.

14. A prokaryotic or eukaryotic host cell capable of expressing the isolated and purified DNA molecule of claim 11.

15. A pharmaceutical composition comprising as an active substance a pharmaceutically effective amount of an isolated and recombinant fusion peptabody of claims 1-10, optionally in combination with pharmaceutically acceptable carriers, diluents and adjuvants.

16. Use of the pharmaceutical composition of claim 15, in the preparation of a medicament for the treatment or prevention of cancer.

17. Use according to claim 16, wherein the cancer is selected from the group consisting of carcinoma, lymphoma, blastoma, sarcoma, liposarcoma, neuroendocrine tumor, mesothelioma, schwanoma, meningioma, adenocarcinoma, melanoma, leukemia, lymphoid malignancy, squamous cell cancer, epithelial squamous cell cancer, lung cancer, small-cell lung cancer, non-small cell lung cancer, adenocarcinoma of the lung, squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, a tumor of the biliary tract, and head and neck cancer.

18. Use according to claim 17, wherein the cancer is head cancer, neck cancer, bladder cancer or melanoma.

19. A kit for treating cancer that expresses epidermal growth factor receptors in a human patient, said kit comprising the isolated and recombinant fusion peptabody of claims 1-10, optionally with reagents and/or instructions for use.

20. The kit of claim 19, further comprising a separate pharmaceutical dosage form comprising an additional anti-cancer agent selected from the group consisting of chemotherapeutic agents, anti-epidermal growth factor receptors antibodies, radioimmunotherapeutic agents, and combinations thereof.

21. A kit for diagnosing cancer that expresses epidermal growth factor receptors in a human patient, said kit comprising the isolated and recombinant fusion peptabody of claims 9-10, optionally with reagents and/or instructions for use.

22. A method for producing the isolated and recombinant fusion peptabody of claims 1-10, comprising the steps of:
a) constructing an isolated and purified DNA molecule encoding the isolated and recombinant fusion peptabody of any one of claims 1-10,
b) allowing expression of said isolated and purified DNA molecule in a cell system under suitable conditions,
c) recovering the isolated and recombinant fusion peptabody.

23. The method of claim 22, **characterized in that** the cell expression system is a prokaryotic cell.

24. The method of claims 22-23, **characterized in that** the suitable conditions consist in culturing the cell expression system at a temperature between 10-40 °C during 2-40 hours.

25. The method of claim 24, **characterized in that** the suitable conditions consist in a temperature of 37°C during 8-16 hours.

26. The method of claims 22-25, **characterized in that** step c) is achieved by extraction of said isolated and recombinant fusion peptabody from the cell expression system subsequently followed by purification and refolding steps.

27. The method of claim 26, **characterized in that** the purification is carried out in the presence of reducing agents and results in the elimination of contamination.

28. The method of claim 26, **characterized in that** the refolding step is carried out by direct dilution in refolding buffer and further comprises serial dialysis.

29. The method of claim 28, **characterized in that** the direct dilution in refolding buffer leads to a final concentration of the isolated and recombinant fusion peptabody below 300 nM.

30. The method of claim 28, **characterized in that** the serial dialysis comprise at least 2 different dialysis buffers.

31. The method of claim 28, **characterized in that** the refolding step consists in the oxidation of the isolated and recombinant fusion peptabody before its concentration.

## Patentansprüche

1. Ein isolierter und rekombinanter Fusionspeptabody, der den epidermalen Wachstumsfaktor ErbB-1, ErbB-3 oder ErbB-4 bindet, umfassend
a) einen Teil eines humanisierten oligomeren Matrixpolypeptids des Knorpels;
b) eine Peptid-Enhancer Sequenz, die eine die Proteinproduktion steigernde Aktivität aufweist und die sich am N-terminalen Ende des Teils des humanisierten oligomeren Matrixpolypeptids des Knorpels befindet und aus der Gruppe bestehend aus YSFE, YSFEDL und YSFEDLYRR ausgewählt ist;
c) einen Teil einer Hinge Region eines Immunoglobulin Polypeptids, das sich am C-terminalen Ende des Teils des humanisierten oligomeren Matrixpolypeptids des Knorpels befindet;
d) einen epidermalen Wachstumsfaktor-Rezeptor Liganden der sich am C terminalen Teil der Hinge Region befindet, umfassend mindestens ein Motiv mit dreidimensionaler Struktur und wobei besagter isolierter und rekombinanter Fusionspeptabody fähig ist den Zelltod in einer Zelle zu induzieren, die den epidermalen Wachstumsfaktor exprimiert.

2. Ein isolierter und rekombinanter Fusionspeptabody gemäss Anspruch 1, wobei der epidermale Wachstumsfaktor ErbB-1 ist.

3. Ein isolierter und rekombinanter Fusionspeptabody gemäss Anspruch 1-2, wobei besagter isolierter und rekombinanter Fusionspeptabody multimer ist.

4. Ein isolierter und rekombinanter Fusionspeptabody gemäss Anspruch 1-3, wobei besagter epidermaler Wachstumsfaktor-Rezeptor Ligand ausgewählt ist aus der Gruppe von:
(a) ein epidermales Wachstumsfaktor Polypeptid oder Fragmente davon,
(b) ein Wachstum-hemmendes Peptid oder Fragmente davon,
(c) ein TGF alpha Polypeptid oder Fragmente davon,
(d) ein Amphiregulin Polypeptid oder Fragmente davon,
e) ein Heparin bindendes epidermaler Wachstumsfaktor ähnliches Polypeptid oder Fragmente davon,
(f) ein Betacellulin Polypeptid oder Fragmente davon.

5. Ein isolierter und rekombinanter Fusionspeptabody gemäss Anspruch 4, wobei besagter epidermaler Wachstumsfaktor-Rezeptor Ligand in vollständiger Sequenzlänge vorhanden ist.

6. Ein isolierter und rekombinanter Fusionspeptabody gemäss Anspruch 1-5, der ausserdem eine Polyhistidin tag Sequenz umfasst.

7. Ein isolierter und rekombinanter Fusionspeptabody gemäss Anspruch 1-6, der ausserdem mindestens eine Effektor-Region umfasst.

8. Ein isolierter und rekombinanter Fusionspeptabody gemäss Anspruch 7, wobei die Effektor-Region ein Zytotoxin umfasst.

9. Ein isolierter und rekombinanter Fusionspeptabody gemäss Anspruch 7, wobei die Effektor Region eine Detektionsgruppierung umfasst.

10. Ein isolierter und rekombinanter Fusionspeptabody gemäss Anspruch 9, wobei die Detektionsgruppierung fluoreszierend ist.

11. Eine isolierte und gereinigte DNA Sequenz, die den isolierten und rekombinanten Fusionspeptabody gemäss einem der Ansprüche 1-7 kodiert.

12. Ein Vektor, der mindestens eine Kopie der isolierten und gereinigten DNA Sequenz gemäss Anspruch 11 umfasst.

13. Der Vektor gemäss Anspruch 12, der ausserdem einen Promotor umfasst, der mit besagtem isoliertem und gereinigtem DNA Molekül operativ verbunden ist.

14. Eine prokariotische oder eukariotische Wirtszelle, die in der Lage ist das isolierte und gereinigte DNA Molekül gemäss Anspruch 11 zu exprimieren.

15. Eine pharmazeutische Zusammensetzung, die als aktive Substanz eine pharmazeutisch wirksame Menge eines isolierten und rekombinanten Fusionspeptabody gemäss Anspruch 1-10 umfasst, gegebenenfalls in Kombination mit pharmazeutisch akzeptablen Trägern, Verdünnungsmitteln und Hilfsstoffen.

16. Verwendung der pharmazeutischen Zusammensetzung gemäss Anspruch 15, zur Herstellung eines Medikaments zur Behandlung oder Prävention von Krebs.

17. Verwendung gemäss Anspruch 16, wobei der Krebs ausgewählt ist aus der Gruppe bestehend aus Karzinom, Lymphom, Blastom, Sarkom, Liposarkom, neuroendocriner Tumor, Mesotheliom, Schwanom, Meningiom, Adenokarzinom, Melanom, Leukämie, lymphoide Malignität, squamöses Karzinom, Plattenepithelkarzinom, Lungenkrebs, kleinzelliger Lungenkrebs, nicht-kleinzelliger Lungenkrebs, Adenokarzinom der Lunge, squamöses Karzinom der Lunge, Peritonealkarzinom, Leberzellenkrebs, Magenkrebs, gastrointestinaler Krebs, Bauchspeicheldrüsenkrebs, Glioblastom, Gebärmutterhalskrebs, Eierstockkrebs, Leberkrebs, Blasenkrebs, Hepatom, Brustkrebs, Darmkrebs, Mastdarmkrebs, Dickdarmkrebs, endometriales oder uterines Karzinom, Speicheldrüsenkrebs, Nierenkrebs, Prostatakrebs, Vulvakrebs, Schilddrüsenkrebs, Leberkarzinom, Analkarzinom, Peniskarzinom, Hodenkrebs, Ösophagus-Krebs, ein Tumor des Gallengangs und Kopf- und Halskrebs.

18. Verwendung gemäss Anspruch 17, wobei der Krebs Kopfkrebs, Halskrebs, Blasenkrebs oder ein Melanom ist.

19. Ein Kit zur Behandlung von Krebs, der epidermale Wachstumsfaktor Rezeptoren in einem menschlichen Patienten exprimiert, wobei der Kit den isolierten und rekombinanten Fusionspeptabody gemäss Anspruch 1-10 umfasst, gegebenenfalls mit Reagenzien und/oder Anweisungen zum Gebrauch.

20. Ein Kit gemäss Anspruch 19, der ausserdem eine separate Verabreichungsform umfasst, umfassend ein weiteres Antikrebsmittel ausgewählt aus der Gruppe bestehend aus chemotherapeutischen Mitteln, Antikörper gegen anti-epidermale Wachstumsfaktor Rezeptoren, radioimmunotherapeutische Mittel sowie Kombinationen davon.

21. Ein Kit zur Diagnose von Krebs, der epidermale Wachstumsfaktor Rezeptoren in einem menschlichen Patienten exprimiert, wobei der Kit den isolierten und rekombinanten Fusionspeptabody gemäss Anspruch 9-10 umfasst, gegebenenfalls mit Reagenzien und/oder Anweisungen zum Gebrauch.

22. Ein Verfahren zur Herstellung eines isolierten und rekombinanten Fusionspeptabody gemäss Anspruch 1-10, wobei das Verfahren die Schritte umfasst:
a) das Herstellen eines isolierten und gereinigten DNA Moleküls, das den isolierten und rekombinanten Fusionspeptabody gemäss einem der Ansprüche 1-10 kodiert,
b) das Ermöglichen der Expression von besagtem isolierten und gereinigten DNA Molekül in ein Zellsystem unter geeigneten Bedingungen,
c) die Gewinnung des isolierten und rekombinanten Fusionspeptabody.

23. Das Verfahren gemäss Anspruch 22, **dadurch gekennzeichnet, dass** das Zellexpressionssystem eine prokariotische Zelle ist.

24. Das Verfahren gemäss Anspruch 22-23, **dadurch gekennzeichnet, dass** die geeigneten Bedingungen darin bestehen, dass das Zellexpressionssystem bei einer Temperatur zwischen 10-40°C während 2-40 Stunden kultiviert wird.

25. Das Verfahren gemäss Anspruch 24, **dadurch gekennzeichnet, dass** die geeigneten Bedingungen eine Temperatur von 37°C während 8-16 Stunden umfassen.

26. Das Verfahren gemäss Ansprüchen 22-25, **dadurch gekennzeichnet, dass** die Verfahrensstufe c) durch Extraktion von besagtem isolierten und rekombinanten Peptabody aus dem Zellextraktionssystem durchgeführt wird, gefolgt von Reinigungsstufen und Rückfaltungsstufen.

27. Das Verfahren gemäss Anspruch 26, **dadurch gekennzeichnet, dass** die Reinigung in Anwesenheit von Reduktionsmittel durchgeführt wird und in der Entfernung von Kontaminationen resultiert.

28. Das Verfahren gemäss Anspruch 26, **dadurch gekennzeichnet, dass** die Rückfaltung durch direkte Verdünnung in Rückfaltungspuffer erfolgt und weiterhin eine serielle Dialyse umfasst.

29. Das Verfahren gemäss Anspruch 28, **dadurch gekennzeichnet, dass** die direkte Verdünnung in Rückfaltungspuffer zu einer Endkonzentration von isoliertem und rekombinanten Fusionspeptabody unter 300nM führt.

30. Das Verfahren gemäss Anspruch 28, **dadurch gekennzeichnet, dass** die serielle Dialyse mindestens zwei unterschiedliche Dialysepuffer umfasst.

31. Das Verfahren gemäss Anspruch 28, **dadurch gekennzeichnet, dass** die Rückfaltungsstufen in der Oxidation des isolierten und rekombinanten Fusionspeptabody vor seiner Konzentration bestehen.

## Revendications

1. Un peptabody de fusion isolé et recombinant, qui se lie au récepteur du facteur de croissance épidermique ErbB-1, ErbB-3 ou ErbB-4, comprenant:
(a) une partie du polypeptide oligomère humanisé de la matrice cartilagineuse;
(b) une séquence d'un peptide enhancer, ayant une activité permettant l'augmentation de la production de protéines, située à l'extrémité N-terminale de la partie du polypeptide oligomère humanisé de la matrice cartilagineuse et choisie dans le groupe constitué par: YSFE, YSFEDL et YSFEDLYRR;
(c) une partie de la région charnière d'un polypeptide d'une immunoglobuline située à l'extrémité C-terminale de la partie du polypeptide oligomère humanisé de la matrice cartilagineuse;
(d) un ligand du récepteur du facteur de croissance épidermique situé à l'extrémité C-terminale de la région charnière, comprenant au moins un motif ayant une structure en trois dimensions,
et dans lequel ledit peptabody de fusion isolé et recombinant est capable d'induire la mort cellulaire d'une cellule exprimant le récepteur du facteur de croissance épidermique.

2. Le peptabody de fusion isolé et recombinant selon la revendication 1, dans lequel le membre du récepteur du facteur de croissance épidermique est ErbB-1.

3. Le peptabody de fusion isolé et recombinant selon les revendications 1-2, dans lequel le peptabody de fusion isolé et recombinant est multimérique.

4. Le peptabody de fusion isolé et recombinant selon les revendications 1-3, dans lequel ledit ligand du récepteur du facteur de croissance épidermique est choisi parmi le groupe de:
(a) un polypeptide du facteur de croissance épidermique ou des fragments de celui-ci,
(b) un peptide du blocage de la croissance ou des fragments de celui-ci,
(c) un polypeptide TGF alpha ou des fragments de celui-ci,
(d) un polypeptide amphiréguline ou des fragments de celui-ci,
(e) un polypeptide du facteur de croissance épidermique EGF-like liant l'héparine ou des fragments de celui-ci,
(f) un polypeptide bêtacelluline ou des fragments de celui-ci.

5. Le peptabody de fusion isolé et recombinant selon la revendication 4, dans lequel ledit ligand du récepteur du facteur de croissance épidermique est présent dans sa séquence complète.

6. Le peptabody de fusion isolé et recombinant selon les revendications 1-5, comprenant en outre une séquence tag polyhistidine.

7. Le peptabody de fusion isolé et recombinant selon les revendications 1-6, comprenant en outre au moins une région effectrice.

8. Le peptabody de fusion isolé et recombinant selon la revendication 7, dans lequel la région effectrice comprend une cytotoxine.

9. Le peptabody de fusion isolé et recombinant selon la revendication 7, dans lequel la région effectrice comprend un élément de détection.

10. Le peptabody de fusion isolé et recombinant selon la revendication 9, dans lequel ledit élément de détection est fluorescent.

11. Une séquence d'ADN isolée et purifiée codant pour le peptabody de fusion isolé et recombinant selon l'une quelconque des revendications 1-7.

12. Un vecteur comprenant au moins une copie de la séquence d'ADN isolée et purifiée selon la revendication 11.

13. Le vecteur de la revendication 12, comprenant de plus un promoteur fonctionnellement lié à ladite molécule d'ADN isolée et purifiée.

14. Une cellule hôte procaryote ou eucaryote capable d'exprimer la molécule d'ADN isolée et purifiée selon la revendication 11.

15. Une composition pharmaceutique comprenant en tant que substance active une quantité efficace sur le plan pharmaceutique du peptabody de fusion isolé et recombinant selon l'une des revendications 1-10, optionnellement en combinaison avec des vehicules, des diluants et des adjuvants acceptables sur le plan pharmaceutique.

16. Utilisation de la composition pharmaceutique selon la revendication 15, pour la préparation d'un médicament dans le traitement ou la prévention du cancer.

17. L'utilisation selon la revendication 16, dans laquelle le cancer est sélectionné parmi le groupe constitué de carcinome, lymphome, blastome, sarcome, liposarcome, tumeur neuroendocrine, mésothéliome, schwanoma, méningiome, adénocarcinome, mélanome, leucémie, cancer lymphoïdien, cancer des cellules squameuses, cancer des cellules épithéliales squameuses, cancer du poumon, cancer du poumon à petites cellules, cancer du poumon non à petites cellules, adénocarcinome du poumon, carcinome squameux du poumon, cancer du péritoine, cancer hépatocellulaire, cancer gastrique ou de l'estomac, cancer gastro-intestinal, cancer du pancréas, glioblastome, cancer du col de l'utérus, cancer de l'ovaire, cancer du foie, cancer de la vessie, hépatome, cancer du sein, cancer du côlon, cancer du rectum, cancer colorectal, carcinome de l'endomètre ou de l'utérus, carcinome des glandes salivaires, cancer rénal ou cancer du rein, cancer de la prostate, cancer de la vulve, cancer de la thyroïde, carcinome hépatique, carcinome anal, carcinome du pénis, cancer des testicules, cancer de l'oesophage, tumeur des voies biliaires, et cancer de la tête et du cou.

18. L'utilisation selon la revendication 17, dans laquelle le cancer est le cancer de la tête, le cancer du cou, le cancer de la vessie ou un mélanome.

19. Un kit de traitement du cancer qui exprime les récepteurs du facteur de croissance épidermique chez un patient humain, ledit kit comprenant le peptabody de fusion isolé et recombinant selon les revendications 1-10, optionnellement avec des réactifs et / ou des instructions d'utilisation.

20. Le kit selon la revendication 19, comprenant de plus une forme distincte de dosage pharmaceutique comprenant un agent anti-cancer supplémentaire choisi parmi le groupe constitué par des agents chimiothérapeutiques, des anticorps anti-récepteurs de facteur de croissance épidermique, des agents radioimmunotherapeutiques, et des combinaisons de ceux-ci.

21. Un kit de diagnostic du cancer qui exprime les récepteurs du facteur de croissance épidermique chez un patient humain, ledit kit comprenant le peptabody de fusion isolé et recombinant selon les revendications 9-10, optionnellement avec des réactifs et / ou des instructions d'utilisation.

22. Une méthode pour produire le peptabody de fusion isolé et recombinant selon les revendications 1-10, comprenant les étapes de:
a) construction des molécules d'ADN isolées et purifiées codant pour le peptabody de fusion isolé et recombinant selon les revendications 1-10,
b) permettant l'expression desdites molécules d'ADN isolées et purifiées dans un système cellulaire dans des conditions appropriées,
c) récupération du peptabody de fusion isolé et recombinant.

23. La méthode selon la revendication 22, **caractérisée en ce que** le système d'expression cellulaire est une cellule procaryote.

24. La méthode selon les revendications 22-23, **caractérisée en ce que** les conditions appropriées consistent à cultiver le système d'expression cellulaire à une température comprise entre 10-40°C pendant 2-40 heures.

25. La méthode selon la revendication 24, **caractérisée en ce que** les conditions appropriées consistent en une température de 37°C pendant 8-16 heures.

26. La méthode selon les revendications 22-25, **caractérisée en ce que** l'étape c) est réalisée par extraction dudit peptabody de fusion isolé et recombinant du système d'expression cellulaire suivi ensuite par une purification et par des étapes de repliement.

27. La méthode selon la revendication 26, **caractérisée en ce que** la purification est effectuée en présence d'agents réducteurs et résulte en l'élimination de contamination.

28. La méthode selon la revendication 26, **caractérisée en ce que** l'étape de repliement est effectuée par dilution directe dans le tampon de repliement et comprend en outre des dialyses en série.

29. La méthode selon la revendication 28, **caractérisée en ce que** la dilution directement dans le tampon de repliement mène à une concentration finale du peptabody de fusion isolé et recombinant inférieure à 300 nM.

30. La méthode selon la revendication 28, **caractérisée en ce que** les dialyses en série comprennent au moins deux tampons de dialyse différents.

31. La méthode selon la revendication 28, **caractérisée en ce que** l'étape de repliement consiste en l'oxydation du peptabody de fusion isolé et recombinant avant sa concentration.


### SEQUENCE LISTING

<110> universite de Lausanne
<120> PEPTABODIES FOR CANCER TREATMENT
<130> 14544/PCT
<140> Not yet known
   <141> 2004-04-05
<150> US 60/460,490
   <151> 2003-04-04
<160> 4
<170> PatentIn version 3.1
<210> 1
   <211> 417
   <212> DNA
   <213> Artificial sequence
<220>
   <223> DNA Sequence Peptabody EGF: MDP01
<400> 1
   atgtatagct ttgaagatct ggctagccat catcatcacc atcatggaga cctgggcccg     60
   cagatgctgc gtgaactgca ggaaaccaac gctgctctgc aggacgttcg tgactacctg    120
   cgtcagctgg ttcgtgaaat caccttcctg aaaaacaccg ttatggaatg cgacgcttgc    180
   ggtatgcagc agactagtcc gcctactccg ccaactccgt ctccgtctac tccgccaact    240
   ccgtctccga gatccaattc tgactctgaa tgcccattgt ctcacgacgg ttactgcttg    300
   cacgacggtg tttgcatgta catcgaagct ctggacaaat acgcttgcaa ctgcgttgtt    360
   ggttacatcg gtgaacgttg ccaataccga gatctgaaat ggtgggaact gcgttaa       417
<210> 2
   <211> 138
   <212> PRT
   <213> Artificial sequence
<220>
   <223> Protein Sequence Peptabody EGF: MDP01
<400> 2
<210> 3
   <211> 333
   <212> DNA
   <213> Artificial sequence
<220>
   <223> DNA Sequence Peptabody GBP: MDP03
<400> 3
   atgtatagct ttgaagatct ggctagccat catcatcacc atcatggaga cctgggcccg 60
   cagatgctgc gtgaactgca ggaaaccaac gctgctctgc aggacgttcg tgactacctg 120
   cgtcagctgg ttcgtgaaat caccttcctg aaaaacaccg ttatggaatg cgacgcttgc 180
   ggtatgcagc agactagtcc gcctactccg ccaactccgt ctccgtctac tccgccaact 240
   ccgtctccga gatctgaaaa cttttccggc ggctgcgtgg cgggctatat gcgtaccccg 300
   gatggccgtt gcaaaccgac cttttatcag taa 333
<210> 4
   <211> 110
   <212> PRT
   <213> Artificial sequence
<220>
   <223> Protein sequence Peptabody GBP : MDP03
<220>
   <221> MISC_FEATURE
   <222> (1)..(110)
   <223>
<400> 4
   Met Tyr Ser Phe Glu Asp Leu Ala Ser His His His His His His Gly
   1               5                   10                  15
   Asp Leu Gly Pro Gln Met Leu Arg Glu Leu Gln Glu Thr Asn Ala Ala
               20                  25                  30
   Leu Gln Asp Val Arg Asp Tyr Leu Arg Gln Leu Val Arg Glu Ile Thr
           35                  40                  45
   Phe Leu Lys Asn Thr Val Met Glu Cys Asp Ala Cys Gly Met Gln Gln
       50                  55                  60
   Thr Ser Pro Pro Thr Pro Pro Thr Pro Ser Pro Ser Thr Pro Pro Thr
   65                  70                  75                  80
   Pro Ser Pro Arg Ser Glu Asn Phe Ser Gly Gly Cys Val Ala Gly Tyr
                   85                  90                  95
   Met Arg Thr Pro Asp Gly Arg Cys Lys Pro Thr Phe Tyr Gln
               100                 105                 110